(19) Europäisches Patentamt
European Patent Office
Office européen des brevets

(11) Veröffentlichungsnummer: **0 334 812 B1**

(12) # EUROPÄISCHE PATENTSCHRIFT

(45) Veröffentlichungstag der Patentschrift: **06.04.94**

(21) Anmeldenummer: **89810208.2**

(22) Anmeldetag: **16.03.89**

(51) Int. Cl.[5]: **C07D 213/81**, C07D 401/12, C07D 413/12, C07D 417/12, C07D 405/12, A01N 47/36, A01N 47/38, A01N 47/34

(54) **Mittel zum Schutz von Pflanzen gegen Krankheiten.**

(30) Priorität: **25.03.88 CH 1141/88**

(43) Veröffentlichungstag der Anmeldung:
**27.09.89 Patentblatt 89/39**

(45) Bekanntmachung des Hinweises auf die Patenterteilung:
**06.04.94 Patentblatt 94/14**

(84) Benannte Vertragsstaaten:
**AT BE CH DE ES FR GB GR IT LI LU NL SE**

(56) Entgegenhaltungen:
**EP-A- 0 004 187**
**CH-A- 614 102**
**US-A- 4 092 421**
**US-A- 4 148 902**

(73) Patentinhaber: **CIBA-GEIGY AG**
**Klybeckstrasse 141**
**CH-4002 Basel(CH)**

(72) Erfinder: **Kunz, Walter, Dr.**
**Buchenstrasse 9**
**CH-4104 Oberwil(CH)**

**EP 0 334 812 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue acylierte Harnstoffderivate der nachstehenden Formel I. Die Erfindung betrifft ferner die Herstellung dieser Substanzen sowie die als Wirkstoff mindestens eine dieser Verbindungen enthaltenden Mittel. Die Erfindung betrifft darüber hinaus die Herstellung der genannten Mittel sowie die Verwendung der Wirkstoffe oder der Mittel zum Schutz von Pflanzen gegen den Befall durch schädliche Mikroorganismen, beispielsweise pflanzenschädigende Pilze, Bakterien und Viren.

Die erfindungsgemässen Verbindungen entsprechen der allgemeinen Formel I

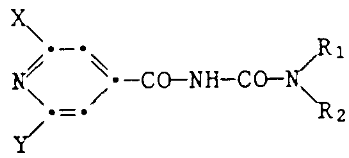

(I)

in welcher bedeuten:

- X Wasserstoff oder Halogen;
- Y Halogen;
- $R_1$, $R_2$ unabhängig voneinander Wasserstoff, $C_1$-$C_6$-Alkyl, mit Halogen, Cyano, $C_1$-$C_4$-Alkoxy oder COO-$C_1$-$C_3$-Alkyl substituiertes $C_1$-$C_6$-Alkyl, $C_3$-$C_5$-Alkenyl, mit Halogen substituiertes $C_3$-$C_5$-Alkenyl, $C_3$-$C_5$-Alkinyl, mit Halogen substituiertes $C_3$-$C_5$-Alkinyl, $C_3$-$C_7$-Cycloalkyl, mit $C_1$-$C_2$-Alkyl, Halogen, Cyano oder COO-$C_1$-$C_3$-Alkyl substituiertes $C_3$-$C_7$-Cycloalkyl, Phenyl, Benzyl, mit $C_1$-$C_3$-Alkyl, $C_1$-$C_3$-Alkoxy oder Halogen substituiertes Phenyl oder Benzyl; und $R_1$ weiterhin auch den Rest Q oder mit $C_1$-$C_3$-Alkyl, $C_3$-$C_7$-Cycloalkyl, $C_1$-$C_3$-Alkoxy oder Halogen substituiertes Q darstellen kann, wobei Q über ein $C_1$-$C_2$-Alkyl an das N-Atom gebunden sein kann; oder worin $R_1$ und $R_2$ zusammen mit dem benachbarten N-Atom einen 3- bis 7-gliedrigen Heterocyclus bilden, der zusätzlich 1 oder 2 weitere Heteroatome, wie O, N oder S, und eine Carbonylgruppe enthalten und mit $C_1$-$C_3$-Alkyl, Halogen oder COO-$C_1$-$C_3$-Alkyl substituiert sein kann;
- Q Furan-2-yl, Thiophen-2-yl, Isoxazol-3-yl, Isoxazol-5-yl, Thiazol-2-yl, 1,2,4-Thiadiazol-3-yl, 1,3,4-Thiadiazol-2-yl, 2-, 3- oder 4-Pyridylreste, mit $C_1$-$C_3$-Alkyl, $C_1$-$C_3$-Alkoxy, $C_1$-$C_3$-Alkylthio oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy, $C_1$-$C_3$-Alkylthio, Halogen oder Cyclopropyl substituierte 2-, 4- oder 5-Pyrimidylreste.

Halogen bedeutet Fluor, Chlor, Brom oder Jod, bevorzugt Chlor und weiter in der Reihenfolge Brom, Fluor und Jod. Als Substituent in einzelnen Resten kann Halogen 1- bis 3-fach vertreten sein.

Unter Alkyl selbst oder als Bestandteil eines anderen Substituenten sind gerad- und verzweigtkettige Alkyle zu verstehen. Sie stellen je nach Zahl der angegebenen Kohlenstoffatome beispielsweise folgende Gruppen dar: Methyl, Ethyl sowie die Isomeren von Propyl, Butyl, Pentyl oder Hexyl, wie z.B. Isopropyl, Isobutyl, tert.-Butyl, sek.-Butyl oder Isopentyl.

Alkenyl steht z.B. für Propenyl-(1), Allyl, Butenyl-(1), Butenyl-(2) oder Butenyl-(3) und Alkinyl bedeutet z.B. Propinyl-(2), Butinyl-(1) oder Pentinyl-(4).

Cycloalkyl bedeutet wahlweise Cyclopropyl, Cyclobutyl, Cyclopentyl, Cyclohexyl oder Cycloheptyl, vorzugsweise Cyclopropyl, Cyclopentyl oder Cyclohexyl.

Die Erfindung bezieht sich insbesondere auf Verbindungen der Formel I, in welcher bedeuten:

- X Wasserstoff oder Halogen;
- Y Halogen;
- $R_1$, $R_2$ unabhängig voneinander Wasserstoff, $C_1$-$C_6$-Alkyl, mit Halogen, Cyano oder COO$C_1$-$C_3$-Alkyl substituiertes $C_1$-$C_6$-Alkyl, $C_3$-$C_5$-Alkenyl, mit Halogen substituiertes $C_3$-$C_5$-Alkenyl, $C_3$-$C_5$-Alkinyl, mit Halogen substituiertes $C_3$-$C_5$-Alkinyl, $C_3$-$C_7$-Cycloalkyl, mit $C_1$-$C_2$-Alkyl, Halogen, Cyano oder COO-$C_1$-$C_3$-Alkyl substituiertes $C_3$-$C_7$-Cycloalkyl, Benzyl, mit $C_1$-$C_3$-Alkyl, $C_1$-$C_3$-Alkoxy oder Halogen substituiertes Benzyl; und $R_1$ weiterhin auch den Rest Q oder mit $C_1$-$C_3$-Alkyl, $C_3$-$C_7$-Cycloalkyl, $C_1$-$C_3$-Alkoxy oder Halogen substituiertes Q darstellen kann, wobei Q über ein $C_1$-$C_2$-Alkyl an das N-Atom gebunden sein kann; oder worin $R_1$ und $R_2$ zusammen mit dem benachbarten N-Atom einen 3- bis 7-gliedrigen Heterocyclus bilden, der zusätzlich 1 oder 2 weitere Heteroatome, wie O, N oder S, und eine Carbonylgruppe enthalten und mit $C_1$-$C_3$-Alkyl, Halogen oder COO-$C_1$-$C_3$-Alkyl substituiert sein kann;

Q Furan-2-yl, Thiophen-2-yl, Isoxazol-3-yl, Isoxazol-5-yl, Thiazol-2-yl, 1,2,4-Thiadiazol-3-yl, 1,3,4-Thiadiazol-2-yl, 2-, 3- oder 4-Pyridylreste, mit $C_1$-$C_3$-Alkyl, $C_1$-$C_3$-Alkoxy, $C_1$-$C_3$-Alkylthio oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy, $C_1$-$C_3$-Alkylthio, Halogen oder Cyclopropyl substituierte 2-, 4- oder 5-Pyrimidylreste.

Aufgrund ihrer besonderen pflanzenschützenden Eigenschaften lassen sich die Verbindungen der Formel I in folgende Gruppen einteilen:

1. Verbindungen der Formel I, worin bedeuten:

X Wasserstoff, Fluor, Chlor oder Brom;

Y Fluor, Chlor oder Brom;

$R_1$ $C_1$-$C_6$-Alkyl, mit Fluor, Chlor, Cyano oder $COOC_1$-$C_3$-Alkyl substituiertes $C_1$-$C_6$-Alkyl, $C_3$-$C_5$-Alkenyl, mit Chlor substituiertes $C_3$-$C_5$-Alkenyl, $C_3$-$C_5$-Alkinyl, mit Chlor substituiertes $C_3$-$C_5$-Alkinyl, $C_3$-$C_7$-Cycloalkyl, mit COO-$C_1$-$C_3$-Alkoxy substituiertes $C_3$-$C_7$-Cycloalkyl, Benzyl, mit $C_1$-$C_3$-Alkoxy oder Halogen substituiertes Benzyl oder Q;

$R_2$ Wasserstoff, $C_1$-$C_6$-Alkyl oder mit Fluor, Chlor, Cyano oder COO-$C_1$-$C_3$-Alkyl substituiertes $C_1$-$C_6$-Alkyl;

$R_1$ und $R_2$ bilden zusammen mit dem benachbarten N-Atom einen 4- bis 7-gliedrigen Heterocyclus, der zusätzlich ein weiteres N-Atom oder ein O-Atom enthalten und mit Methyl oder COO-$C_1$-$C_3$-Alkyl substituiert sein kann;

Q Furan-2-yl, Thiophen-2-yl, Isoxazol-3-yl oder Isoxazol-5-yl, 2-, 3- oder 4-Pyridylreste, mit $C_1$-$C_3$-Alkyl, $C_1$-$C_3$-Alkoxy oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy oder Halogen substituierte 2-, 4- oder 5-Pyrimidylreste.

2. Verbindungen der Formel I, worin bedeuten:

X Wasserstoff, Fluor, Chlor oder Brom;

Y Fluor, Chlor oder Brom;

$R_1$ $C_1$-$C_6$-Alkyl, mit Halogen substituiertes $C_1$-$C_6$-Alkyl oder Q;

$R_2$ Wasserstoff, $C_1$-$C_6$-Alkyl oder mit Fluor, Chlor, Cyano oder COO-$C_1$-$C_3$-Alkyl substituiertes $C_1$-$C_6$-Alkyl;

$R_1$ und $R_2$ bilden zusammen mit dem benachbarten N-Atom einen 4- bis 7-gliedrigen Heterocyclus, der zusätzlich ein N- oder ein O-Atom enthalten und mit Halogen substituiert sein kann;

Q Thiazol-2-yl, 1,2,4-Thiazol-3-yl, 1,3,4-Thiadiazol-2-yl, 2-, 3- oder 4-Pyridylreste oder mit $C_1$-$C_3$-Alkyl, $C_1$-$C_3$-Alkoxy oder Halogen substituierte 2-, 3- oder 4-Pyridylreste.

Von den vorgenannten Verbindungsgruppen sind folgende Untergruppen bevorzugt:

1.1 Verbindungen der Formel I, worin bedeuten:

X Wasserstoff, Chlor oder Brom;

Y Chlor oder Brom;

$R_1$ $C_1$-$C_4$-Alkyl, mit Chlor oder COO-$C_1$-$C_3$-Alkyl substituiertes $C_1$-$C_4$-Alkyl, $C_3$-$C_5$-Alkenyl, $C_3$-$C_5$-Alkinyl, Cyclopropyl, mit $COOCH_3$ substituiertes Cyclopropyl, Benzyl, mit Methoxy oder Chlor substituiertes Benzyl oder Q;

$R_2$ Wasserstoff, $C_1$-$C_4$-Alkyl, mit Chlor oder COO-$C_1$-$C_3$-Alkyl substituiertes $C_1$-$C_4$-Alkyl;

$R_1$ und $R_2$ bilden zusammen mit dem benachbarten N-Atom einen 5- oder 6-gliedrigen Heterocyclus, der zusätzlich ein weiteres N- oder ein O-Atom enthalten und mit Methyl oder COO-$C_1$-$C_3$-Alkyl substituiert sein kann;

Q Furan-2-yl, Thiophen-2-yl, Isoxazol-3-yl, Isoxazol-5-yl, 2-, 3- oder 4-Pyridylreste, mit $C_1$-$C_3$-Alkyl, $C_1$-$C_3$-Alkoxy, Chlor oder Brom substituierte 2-, 3- oder 4-Pyridylreste; 2-, 4- oder 5-Pyrimidylreste oder mit $C_1$-$C_3$-Alkyl, $C_1$-$C_3$-Alkoxy, Chlor oder Brom substituierte 2-, 4- oder 5-Pyrimidylreste.

2.1 Verbindungen der Formel I, worin bedeuten:

X Wasserstoff, Chlor oder Brom;

Y Chlor oder Brom;

$R_1$ $C_1$-$C_4$-Alkyl, mit Fluor oder Chlor substituiertes $C_1$-$C_4$-Alkyl oder Q;

$R_2$ Wasserstoff, $C_1$-$C_4$-Alkyl, mit Chlor oder COO-$C_1$-$C_3$-Alkyl substituiertes $C_1$-$C_4$-Alkyl;

$R_1$ und $R_2$ bilden zusammen mit dem benachbarten N-Atom einen 5- oder 6-gliedrigen Heterocyclus, der zusätzlich ein weiteres N-Atom oder ein O-Atom enthalten und mit Chlor oder Brom substituiert sein kann;

Q Thiazol-2-yl, 1,2,4-Thiadiazol-3-yl, 1,3,4-Thiadiazol-2-yl, 2-, 3- oder 4-Pyridylreste oder

mit Methyl, Methoxy, Fluor, Chlor oder Brom substituierte 2-, 3- oder 4-Pyridylreste.

Die folgenden Verbindungen zeichnen sich durch besonders vorteilhafte pflanzenschützende Eigenschaften aus:

N-(2,6-Dichlorisonicotinoyl)-carbamoyl-piperidin;

N,N-Dimethyl-N'-(2,6-dichlorisonicotinoyl)-harnstoff.

Substituierte acylierte Harnstoffderivate sind bereits bekannt, so sind z.B. in der USA-Patentschrift No. 4,148,902 Phenylaminocarbonyl-pyridincarboxamide als Insektizide beschrieben. Verbindung 2,6-Dichloro-N-(((4-chlorophenyl)amino)carbonyl)-4-pyridincarboxamid (aus US-4,148,902) ist vorbekannt und bildet keinen Gegenstand der vorliegenden Erfindung.

Es wurde nun überraschenderweise gefunden, dass die erfindungsgemässen Verbindungen der Formel I durch ihre Verwendung den Befall von Pflanzen durch schädliche Mikroorganismen verhindern und damit den befallsbedingten Schädigungen der Pflanzen vorbeugen. Für die erfindungsgemässen Wirkstoffe ist charakteristisch, dass der Schutz der Pflanzen sowohl durch direkte Einwirkung auf die pflanzenschädigenden Mirkoorganismen mittels Blattapplikation (direkte Wirkung) oder Bodenapplikation (systemische Wirkung) als auch durch Aktivierung und Stimulierung des pflanzeneigenen Abwehrsystems (Immunisierung) eintreten kann. Der grosse Vorteil der Verbindungen der Formel I besteht darin, dass die Gesunderhaltung der mit diesen Stoffen behandelten Pflanzen auch aus eigener Kraft ohne Einsatz weiterer mikrobizider Substanzen während der Vegetationsperiode gewährleistet werden kann. Demzufolge ist es möglich, durch die Anwendung der erfindungsgemässen Wirkstoffe nachteilige Nebeneffekte, wie sie bei der direkten Parasitenbekämpfung mit chemischen Substanzen z.B. einerseits durch Schädigung der Nutzpflanzen (Phytotoxizität) und andererseits durch Hervorrufung von Resistenzerscheinungen bei den schädlichen Mikroorganismen in Erscheinung treten können, zu vermeiden, was vorteilhafterweise ein völlig ungestörtes Wachstum der Nutzpflanzen zur Folge hat.

Aufgrund der doppelten Wirkungsweise der erfindungsgemässen Verbindungen der Formel I, nämlich einerseits der direkten Bekämpfung der Pflanzenpathogene und andererseits der Erhöhung der allgemeinen Abwehrbereitschaft der mit diesen Wirkstoffen behandelten Pflanzen durch Immunisierung kann ein breit gefächerter Schutz der Pflanzen gegen Krankheiten erzielt werden. Die Anwendung der erfindungsgemässen Wirkstoffe ist deshalb besonders für praktische Bedingungen geeignet. Darüber hinaus bewirkt die den Verbindungen der Formel I zueigene systemische Aktivität, dass sich der Schutzeffekt auch auf zuwachsende Pflanzenteile der behandelten Pflanzen erstreckt.

Die allgemein pflanzenschützende Aktivität der erfindungsgemässen Wirkstoffe ist z.B. gegen die den folgenden Klassen angehörenden phytopathogenen Fungi wirksam: Fungi imperfecti (z.B. Botrytis, Helminthosporium, Fusarium, Septoria, Cercospora und Alternaria); Basidiomyceten (z.B. die Gattungen Hemileia, Rhizoctonia, Puccinia); Ascomyceten (z.B. Venturia, Podosphaera, Erysiphe, Monilinia, Uncinula).

Darüber hinaus können die Wirkstoffe besonders vorteilhaft gegen folgende Schadorganismen eingesetzt werden:

Pilze, wie z.B. Oomyceten (z.B. Plasmopara viticola, Phytophthora infestans, Peronospora tabacina, Pseudoperonospora), Fungi imperfecti (z.B. Colletotrichum lagenarium, Pyricularia oryzae, Cercospora nicotinae), Ascomyceten (z.B. Venturia inaequalis);

Bakterien, wie z.B. Pseudomonaden (Pseudomonas lachrymans, Pseudomonas tomato, Pseudomonas tabaci); Xanthomonaden (z.B. Xanthomonas oryzae, Xanthomonas vesicatoria); Erwinia (z.B. Erwinia amylovora); und Viren, wie z.B. das Tabakmosaikvirus.

Die erfindungsgemässen Verbindungen können zum Schutz von Pflanzen unterschiedlicher Nutzkulturen eingesetzt werden.

Für den Einsatz der erfindungsgemässen Verbindungen der Formel I im Rahmen der Erfindung sind beispielsweise folgende Pflanzenarten geeignet: Getreide (Weizen, Gerste, Roggen, Hafer, Reis, Sorghum und Verwandte); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Als besonders geeignete Zielkulturen für die Anwendung des erfindungsgemässen Verfahrens sind folgende Pflanzen anzusehen: Gurke, Tabak, Reben, Reis, Pfeffer, Kartoffeln, Tomate, Weizen, Gerste, Birne und Apfel.

Die Verbindungen der Formel I werden hergestellt, indem man umsetzt:
1) ein Isonicotinsäureamid der Formel II

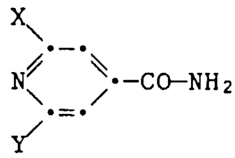

(II)

mit

a) einem Carbamoylhalogenid der Formel IIIa

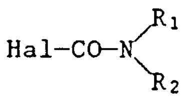

(IIIa)

oder

b) einem Carbamoylazolid der Formel IIIb

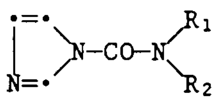

(IIIb)

oder zur Herstellung der Verbindungen der Formel I, in welchen $R_1$ oder $R_2$ Wasserstoff darstellt,

c) mit einem Isocyanat der Formel IIIc

O = C = N-A (IIIc)

in Gegenwart einer Base in aprotischen Lösungsmitteln, wobei in den vorstehend genannten Verbindungen Hal Halogen, bevorzugt Chlor, darstellt, A die Bedeutung von $R_1$ bzw. $R_2$ hat und dieser Rest sowie X und Y die unter Formel I angegebenen Bedeutungen haben.

Die Reaktionen gemäss den Verfahrensvarianten 1a, 1b und 1c finden bei Temperaturen von 0° bis 180°C, bevorzugt 20° bis 120°C, statt.

Als Basen kommen organische und anorganische Basen in Betracht, z.B. tertiäre Amine wie Trialkylamine (Trimethylamin, Triethylamin, Tripropylamin usw.), Pyridin, Pyridinbasen (4-Dimethylaminopyridin, 4-Pyrrolidylaminopyridin,Collidin), Oxide und Hydroxide, Carbonate und Hydrogencarbonate von Alkali- und Erdalkalimetallen sowie Alkaliacetate.

Als Reaktionsmedien in Anpassung an die jeweiligen Reaktionsbedingungen werden geeignete reaktionsinerte Lösungs- und Verdünnungsmittel verwendet. Als Beispiele sind zu nennen: aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Ketone wie Aceton, Diethylketon, Methylethylketon; sowie Gemische solcher Lösungsmittel untereinander.

2. Nach einem weiteren Verfahren zur Herstellung der Verbindungen der Formel I kann man eine Verbindung der Formel II

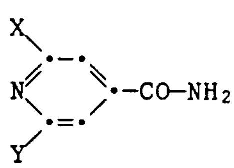

(II)

a) mit $(COCl)_2$ oder $CO(Cl)_2$ zu einem Isocyanat der Formel IV

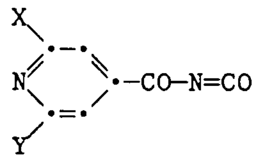

(IV),

reagieren lassen, welches anschliessend
b) mit einem Amin der Formel V

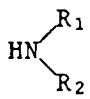

(V)

umgesetzt wird.

Die Reaktion gemäss 2a findet bei Temperaturen von -20° bis 260°C in inerten Lösungsmitteln statt.

Der anschliessende Reaktionsschritt gemäss 2b wird bei Temperaturen von -40° bis 100°C, bevorzugt -10° bis 50°C, vorzugsweise in Gegenwart katalytischer Mengen einer Base, z.B. eines tertiären Amins, wie Trimethylamin, Triethylamin oder Diazabicyclooctan, in inerten Lösungsmitteln durchgeführt.

Als Reaktionsmedien für die Reaktionsschritte 2a und 2b kommen z.B. folgende Lösungsmittel in Frage: Ether, wie Diethylether, Diisopropylether, Tetrahydrofuran oder Dioxan, halogenierte Kohlenwasserstoffe, wie Ethylenchlorid, Methylenchlorid, Trichlorethan, Chloroform, und Kohlenwasserstoffe, wie Toluol oder Xylol.

Die Herstellung der Ausgangsstoffe ist dem Fachmann geläufig und aus der Literatur bekannt.

So lassen sich die Amide der Formel II durch Umsetzung eines Isonicotinsäureesters, Isonicotinsäurehalogenids oder Isonicotinsäureazolids mit Ammoniak in einem Lösungsmittel wie Wasser, Tetrahydrofuran, Toluol, Acetonitril oder einem Gemisch davon, herstellen.

Die vorgenannten Ester und Halogenide sind bekannt oder lassen sich nach bekannten Methoden herstellen (vgl. Houben-Weyl 5/3, 925). Die Azolide werden z.B. entweder durch Umsetzung von Isonicotinsäurehalogenide mit Azolen oder durch Umsetzung von Isonicotinsäure mit Dicarbonyldiimidazol hergestellt (vgl. Angew. Chemie 1962, 409-411).

Ein vortheilhaftes Verfahren zur Herstellung von Isonicotinsäureamiden der Formel II ist die Umsetzung des Säurehalogenids mit Hexamethyldisilazan der Formel $[(CH_3)_2Si]_2NH$ oder einem analogen Silylamin in einem inerten Lösungsmittel, wie Dichlormethan oder Trichlorethan, bei Temperaturen von -10° bis 80°C, bevorzugt 0° bis 40°C und anschliessender Hydrolyse z.B. mit einem Alkohol, wie Methanol, und verdünnter Mineralsäure, wie z.B. Schwefelsäure (vgl. Synthetic Communications 1985, 519).

Die im Rahmen der Erfindung zur Anwendung gelangenden mikrobiziden Mittel zum Schutz von Pflanzen gegen Krankheiten, welche die Verbindungen der Formel I als Aktivstoffe enthalten, sind als Teil der Erfindung zu betrachten.

Wirkstoffe der Formel I werden üblicherweise in Form von Zusammensetzungen verwendet und können gleichzeitig oder nacheinander mit weiteren Wirkstoffen auf die Pflanze oder deren Umgebung gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein Verfahren zur Anwendung eines Wirkstoffes der Formel I bzw. eines agrochemischen Mittels, das mindestens einen dieser Wirkstoffe enthält, ist das Aufbringen auf die Pflanze (Blattapplikation). Die Wirkstoffe der Formel I können aber auch über den Erdboden durch das Wurzelwerk in die Pflanze gelangen (Bodenapplikation), indem man den Standort der Pflanze mit einer flüssigen Zubereitung tränkt oder die Substanzen in fester Form in den Boden einbringt z.B. in Form von Granulat. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung

beschichtet (Beizapplikation). Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt. Zu diesem Zweck werden sie z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt bei 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden hergestellt durch inniges Vermischen und/oder Vermahlen der Wirkstoffe mit Streckmitteln, wie z.B. mit Lösungsmitteln, festen Trägerstoffen, und gegebenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid; sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I nicht-ionogene, kation-und/oder anionaktive Tenside mit guten Emulgier-, Dispergier- und Netzeigenschaften in Betracht. Unter Tensiden sind auch Tensidgemische zu verstehen.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedere Hydroxyalkylreste aufweisen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure oder von natürlichen Fettsäuregemischen, die z.B. aus Kokosnuss- oder Talgöl gewonnen werden können, genannt.

Als synthetische Tenside können insbesondere Fettalkoholsulfonate, Fettalkoholsulfate, sulfonierte Benzimidazolderivate oder Alkylsulfonate Verwendung finden. Die Fettalkoholsulfonate oder -sulfate liegen in der Regel als Alkali-, Erdalkali- oder gegebenenfalls substituierte Ammoniumsalze vor und weisen einen Alkalirest mit 8 bis 22 C-Atomen auf.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-% insbesondere 99,8 bis 5 Gew.-% eines festen oder flüssigen Zusatzstoffes und 0 bis 25 Gew.-%, insbesondere 0,1 bis 25 Gew.-% eines Tensides.

Die nachfolgenden Beispiele dienen zur näheren Erläuterung der Erfindung.

1. Herstellungsbeispiele

Beispiel 1.1:

a) Herstellung von 2,6-Dichlorisonicotinsäureamid (Ausgangsmaterial)

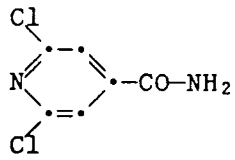

53,1 g Hexamethyldisilazan werden in 600 ml abs. Methylenchlorid vorgelegt. Unter Kühlen werden bei 0-5°C 63 g 2,6-Dichlorisonicotinsäurechlorid in 500 ml Methylenchlorid zugetropft und über Nacht bei Raumtemperatur weitergerührt. Anderntags werden nacheinander 60 ml Methanol und 600 ml 5%ige Schwefelsäure zugetropft. Der ausgefallene Niederschlag wird abfiltriert und mit Wasser gewaschen. Man erhält 54 g (95 %) weisse Kristalle vom Smp. 198-200°C.

b) Herstellung von 2,6-Dichlorisonicotinoyl-isocyanat (Zwischenprodukt)

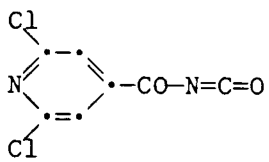

18 g 2,6-Dichlorisonicotinsäureamid werden in 180 ml Ethylenchlorid gelöst und bei 0-5°C tropfenweise mit 14,7 g Oxalylchlorid versetzt. Anschliessend wird während 8 Std. Unter Rückfluss gekocht, abgekühlt und die Lösung eingedampft. Hochvakuumdestillation ergibt ein bei 65-7°C/4 Pa siedendes Oel (82,1 % der Theorie).

c) Herstellung von N-(2,6-Dichlorisonicotinoyl)-carbamoyl-piperidin (Endprodukt)

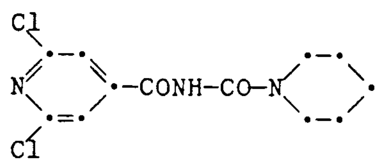

0,595 g Piperidin in 15 ml abs. Toluol werden bei Raumtemperatur tropfenweise mit der Lösung von 1,51 g des unter a) erhaltenen Isocyanats in 5 ml Toluol versetzt. Nach Abklingen der exothermen Reaktion wird noch während 5 Std. bei Raumtemperatur weitergerührt und anschliessend der gebildete Niederschlag abfiltriert. Nach Umkristallisierung aus Aceton erhält man weisse Kristalle, Smp. 183-185°C.

Gemäss den vorstehend beschriebenen Herstellungsweisen werden die nachfolgend aufgeführten Verbindungen erhalten.

Tabelle 1

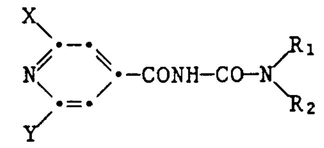

| Verb. Nr. | X | Y | $R_1$ | $R_2$ | physik. Daten |
|---|---|---|---|---|---|
| 1.1 | Cl | Cl | $CH_3$ | $CH_3$ | Smp. 171-178°C |
| 1.2 | Cl | Cl | $C_2H_5$ | $C_2H_5$ | Smp. 135-137°C |
| 1.3 | Cl | Cl | $C_3H_7$(i) | $C_3H_7$(i) | |
| 1.4 | Cl | Cl | $C_4H_9$(n) | $C_4H_9$(n) | Smp. 81-84°C |
| 1.5 | Cl | Cl | $C_4H_9$(sec) | $C_4H_9$(sec) | |
| 1.6 | Cl | Cl | H | $CH_3$ | Smp. 264-266°C |
| 1.7 | Cl | Cl | H | $C_3H_7$(i) | |
| 1.8 | Cl | Cl | H | $C_4H_9$(n) | Smp. 149-150°C |
| 1.9 | Cl | Cl | H | $C_4H_9$(t) | Smp. 191-193°C |
| 1.10 | Cl | Cl | H | $C_6H_{13}$(n) | |
| 1.11 | Cl | Cl | H | cyclo-Propyl | Smp. 221-223°C |
| 1.12 | Cl | Cl | H | cyclo-Pentyl | Smp. 181-184°C |
| 1.13 | Cl | Cl | H | cyclo-Hexyl | |
| 1.14 | Cl | Cl | H | $CH_2=CH-CH_2$ | |
| 1.15 | Cl | Cl | H | $CH_2C\equiv CH$ | |
| 1.16 | Cl | Cl | H | $CH_2CN$ | |
| 1.17 | Cl | Cl | H | $CH_2CH_2CN$ | |
| 1.18 | Cl | Cl | $CH_2CN$ | $CH_2CN$ | Smp. 135-141°C |
| 1.19 | Cl | Cl | $CH_2CH_2CN$ | $CH_2CH_2CN$ | Smp. 165-166°C |
| 1.20 | Cl | Cl | $-(CH_2)_5-$ | | Smp. 183-185°C |
| 1.21 | Cl | Cl | $-(CH_2)_4-$ | | |
| 1.22 | Cl | Cl | $-(CH_2)_2-CO-(CH_2)_2-$ | | |
| 1.23 | Cl | Cl | $-(CH_2)_6$ | | |
| 1.24 | Cl | Cl | $-(CH_2)O(CH_2)_2-$ | | Smp. 195-198°C |
| 1.25 | Cl | Cl | $CH_3$ $CH_3$<br>$-CH_2-CH-O-CH-CH_2-$ | | Smp. 200-202°C |
| 1.26 | Cl | Cl | $-CH_2-CH_2-S-CH_2-CH_2-$ | | |
| 1.27 | Cl | Cl | $-CH_2-CH_2-N(CH_3)CH_2-CH_2-$ | | |

Tabelle 1 (Fortsetzung)

| Verb. Nr. | X | Y | $R_1$ | $R_2$ | physik. Daten |
|---|---|---|---|---|---|
| 1.28 | Br | Br | $-(CH_2)_5-$ | | |
| 1.29 | Br | Br | $-(CH_2)_4-$ | | |
| 1.30 | Br | Br | $-(CH_2)_7-$ | | |
| 1.31 | Br | Br | $-CH_2-CH_2-O-CH_2-CH_2-$ | | |
| 1.32 | Cl | H | $-(CH_2)_5-$ | | |
| 1.33 | Cl | H | $-(CH_2)_4-$ | | |
| 1.34 | Cl | H | $-(CH_2)_7-$ | | |
| 1.35 | F | F | $-(CH_2)_5-$ | | |
| 1.36 | J | J | $-(CH_2)_5-$ | | |
| 1.37 | Cl | H | $-(CH_2)_5-$ | | |
| 1.38 | Cl | H | $-(CH_2)_6-$ | | |
| 1.39 | Cl | H | 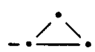 | H | |
| 1.40 | Cl | H | 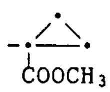 | H | |
| 1.41 | Br | Br | $CH_2-C\equiv CH$ | H | |
| 1.42 | Br | Br | $CH_2-C\equiv CH_2$ | H | |
| 1.43 | Br | Br | Cyclohexyl | H | |
| 1.44 | Br | Br | Cyclopropyl | H | |
| 1.45 | Br | Br | 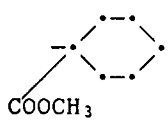 | H | |
| 1.46 | Br | Br | $CH_3$ | $CH_3$ | |
| 1.47 | Br | Br | $C_4H_9$(sec) | $C_4H_9$(sec) | |
| 1.48 | Br | Br | Benzyl | H | |
| 1.49 | Cl | H | 2-Pyridyl | H | |
| 1.50 | F | F | 3-Pyridyl | H | |
| 1.51 | F | F | 4-Pyridyl | H | |
| 1.52 | Cl | Cl | Benzyl | Me | |
| 1.53 | Cl | Cl | $2,6-(CH_3)_2-C_6H_3$ | H | |
| 1.54 | Cl | Cl | $3,5-(Cl)_2-C_6H_3$ | H | Smp. 247-249°C |
| 1.55 | Cl | Cl | $2-F-C_6H_4$ | H | |

Tabelle 1 (Fortsetzung)

| Verb. Nr. | X | Y | $R_1$ | $R_2$ | physik. Daten |
|---|---|---|---|---|---|
| 1.56 | Cl | Cl | 2-F-Benzyl | H | |
| 1.57 | Cl | Cl | 4-Cl-Benzyl | H | |
| 1.58 | Br | Br | $C_4H_9(n)$ | $C_4H_9(n)$ | |
| 1.59 | Br | Br | $CH_2CH_2-CN$ | H | |
| 1.60 | F | F | $CH_2-CN$ | $CH_2CN$ | |
| 1.61 | F | F | $CH_2-CN$ | H | |
| 1.62 | Cl | Cl | $2-Cl-C_6H_4$ | H | Smp. 244-247°C |
| 1.63 | Cl | Cl | 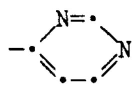 | H | |
| 1.64 | Br | Br | 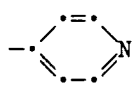 | H | |
| 1.65 | Cl | Cl | 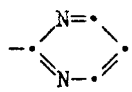 | H | Smp. 205-207°C |
| 1.66 | Cl | Cl | 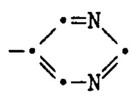 | H | |
| 1.67 | Cl | Cl | 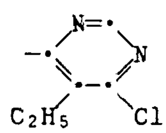 | H | Smp. 177-179°C |
| 1.68 | Cl | Cl | 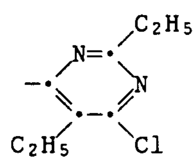 | H | |
| 1.69 | Cl | Cl | 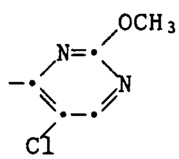 | H | |
| 1.70 | Cl | Cl | 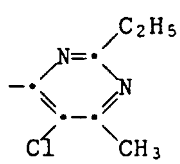 | H | |

Tabelle 1 (Fortsetzung)

| Verb. Nr. | X | Y | $R_1$ | $R_2$ | physik. Daten |
|---|---|---|---|---|---|
| 1.71 | Cl | Cl | 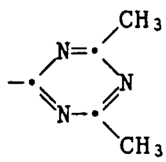 | H | |
| 1.72 | Cl | Cl | 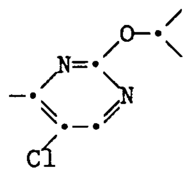 | H | |
| 1.73 | Cl | Cl | 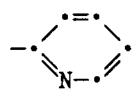 | $CH_3$ | |
| 1.74 | Cl | Cl | 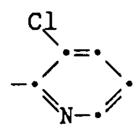 | H | |
| 1.75 | Cl | Cl | 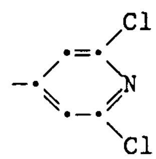 | H | |
| 1.76 | Cl | Cl | $-CH(OC_2H_5)CN$ | H | |
| 1.77 | Cl | Cl | 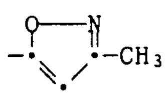 | H | Smp. 180-182°C |
| 1.78 | Cl | Cl | $CH_2-CH=CH_2$ | $CH_2-CH=CH_2$ | |
| 1.79 | Cl | Cl | $CH_2C\equiv CJ$ | H | |
| 1.80 | Cl | Cl | 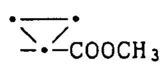 | H | |
| 1.81 | Cl | Cl | 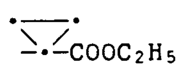 | H | |
| 1.82 | Cl | Cl | 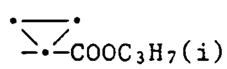 | H | |
| 1.83 | Cl | Cl | 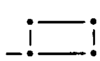 | H | |

Tabelle 1 (Fortsetzung)

| Verb. Nr. | X | Y | $R_1$ | $R_2$ | physik. Daten |
|---|---|---|---|---|---|
| 1.84 | Cl | Cl | 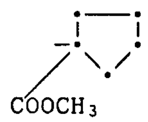 | H | |
| 1.85 | Cl | Cl | 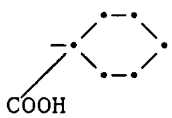 | H | |
| 1.86 | Cl | Cl | Cyclohexyl | H | |
| 1.87 | Cl | Cl | 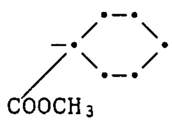 | H | |
| 1.88 | Cl | Cl | Cycloheptyl | H | |
| 1.89 | Cl | Cl | Benzyl | H | Smp. 215-217°C |
| 1.90 | Cl | Cl | 1,2,4,-Thiadiazol-3-yl | H | |
| 1.91 | Cl | Cl | 1,3,4,-Thiadiazol-2-yl | H | Smp. 250°C (Zers.) |
| 1.92 | Cl | Cl | Thiazol-2-yl | H | |
| 1.93 | Cl | Cl | 2-Pyridyl | H | Smp. 207-209°C |
| 1.94 | Cl | Cl | 3-Pyridyl | H | |
| 1.95 | Cl | Cl | 4-Pyridyl | H | |
| 1.96 | Cl | Cl | 2-Pyridyl | $CH_3$ | |
| 1.97 | Cl | Cl | Thiophen-2-yl | $CH_3$ | |
| 1.98 | Cl | Cl | 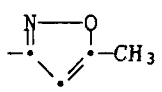 | H | Smp. 193-195°C |
| 1.99 | Br | Br | 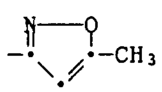 | H | |
| 1.100 | Cl | H | 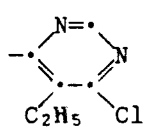 | H | |
| 1.101 | Br | Br | 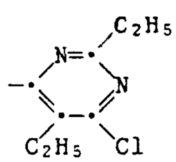 | H | |

Tabelle 1 (Fortsetzung)

| Verb. Nr. | X | Y | $R_1$ | $R_2$ | physik. Daten |
|---|---|---|---|---|---|
| 1.102 | J | J | 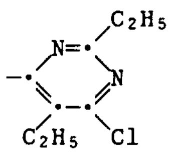 | H | |
| 1.103 | F | F | 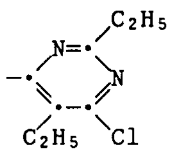 | H | |
| 1.104 | Cl | Cl | 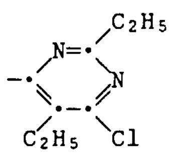 | $CH_3$ | |
| 1.105 | Cl | Cl | 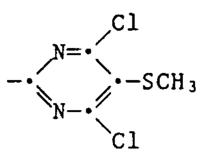 | H | |

Tabelle 2

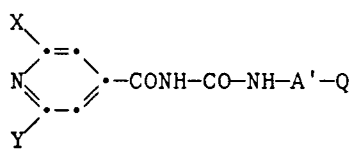

| Verb. Nr. | X | Y | A' | Q | physik. Daten |
|---|---|---|---|---|---|
| 2.1 | Cl | Cl | $-CH_2-$ | 3-Pyridyl | |
| 2.2 | Cl | H | $-CH_2-CH_2-$ | 4-Pyridyl | |
| 2.3 | Cl | Cl | $-CH_2-$ | 2-Furyl | Smp. 188-189°C |
| 2.4 | Cl | Cl | $-CH_2-$ | 2-Thienyl | Smp. 188-190°C |
| 2.5 | Cl | Cl | $-CH_2-$ | 2-Pyridyl | Smp. 222-224°C |
| 2.6 | Cl | Cl | $-CH_2-$ | 4-Pyridyl | |
| 2.7 | Cl | Cl | $-CH_2CH_2-$ | 2-Furyl | |
| 2.8 | Cl | Cl | $-CH(CH_3)-$ | 2-Furyl | |

Tabelle 2 (Fortsetzung)

| Verb. Nr. | X | Y | A' | Q | physik. Daten |
|---|---|---|---|---|---|
| 2.9 | Br | Br | $-CH(CH_3)-$ | 2-Pyridyl | |
| 2.10 | F | F | $-CH_2-$ | 2-Furyl | |
| 2.11 | Cl | Cl | $-CH(CH_3)-$ | 2-Furyl | |

Tabelle 3 (Zwischenprodukte der Formel IV)

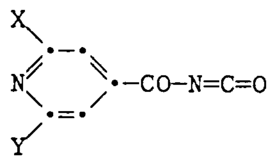

| Verb. Nr. | X | Y | physik. Daten |
|---|---|---|---|
| 3.1 | Cl | Cl | Sdp. 65-67°C/4 Pa |
| 3.2 | Br | Br | |
| 3.3 | Cl | H | |
| 3.4 | F | F | |
| 3.5 | J | J | |

2. Formulierungsbeispiele für Wirkstoffe der Formel I (% = Gewichtsprozent)

2.1 Spritzpulver

| | a) | b) | c) |
|---|---|---|---|
| Wirkstoff aus den Tabellen | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

2.2 Emulsions-Konzentrat

| | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

2.3 Stäubemittel

| | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

2.4 Extruder Granulat

| | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.5 Umhüllungs-Granulat

| | |
|---|---|
| Wirkstoff aus den Tabellen | 3 % |
| Polyethylenglykol (MG 200) | 3 % |
| Kaolin (MG = Molekulargewicht) | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.6 Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Colletotrichum lagenarium auf Cucumis sativus L.

a) Gurkenpflanzen werden nach 2-wöchiger Anzucht mit einer aus Spritzpulver des Wirkstoffes hergestellten Spritzbrühe besprüht (Konzentration: 200 ppm). Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22 °C bis 23 °C weitergeführt.

Die Beurteilung der Schutzwirkung erfolgt aufgrund des Pilzbefalls 7-8 Tage nach der Infektion.

b) Gurkenpflanzen werden nach 2-wöchiger Anzucht mit einer aus Spritzpulver des Wirkstoffes hergestellten Spritzbrühe durch Bodenapplikation behandelt (Konzentration: 60 oder 20 ppm bezogen auf das Bodenvolumen). Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22 °C weitergeführt.

Die Beurteilung der Schutzwirkung erfolgt aufgrund des Pilzbefalls 7-8 Tage nach der Infektion.

c) Gurkenpflanzen werden nach 2-wöchiger Anzucht mit einer aus Spritzpulver des Wirkstoffes hergestellten Spritzbrühe besprüht (Konzentration: 200 ppm).

Nach 3 Wochen werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchigkeit und bei 22 ° bis 23 °C weitergeführt.

Die Beurteilung der Schutzwirkung erfolgt aufgrund des Pilzbefalls 7-8 Tage nach der Infektion.

Verbindungen aus den Tabellen 1 und 2 zeigten in den Tests gute Wirkung. So reduzierte z.B. die Verbindung 1.1, 1.77 oder 1.98 den Pilzbefall auf 0 bis 20 %. Unbehandelte jedoch infizierte Kontrollpflanzen wiesen dagegen einen Colletotrichum-Befall von 100 % auf.

Beispiel 3.2: Wirkung gegen Puccinia graminis auf Weizen

a) Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 °C werden die infizierten Pflanzen in einem Gewächshaus bei ca. 22 °C aufgestellt. Die Beurteilung der Rostpustelnentwicklung erfolgt 12 Tage nach der Infektion.

b) Zu Weizenpflanzen wird 5 Tage nach Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 °C werden die infizierten Pflanzen in einem Gewächshaus bei ca. 22 °C aufgestellt. Die Beurteilung der Rostpustelnentwicklung erfolgt 12 Tage nach der Infektion.

Verbindungen aus den Tabellen 1 und 2 zeigten gegen Puccinia-Pilze eine gute Wirkung. So reduzierten z.B. die Verbindungen 1.1, 1.18, 1.24 und 1.25 den Pilzbefall auf 0 bis 20 %. Unbehandelte jedoch infizierte Kontrollpflanzen wiesen dagegen einen Puccinia-Befall von 100 % auf.

Beispiel 3.3: Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgte nach einer Inkubation der infizierten Pflanzen während 5 Tagen bei 9O-100 % relativer Luftfeuchtigkeit und 20 °C.

b) Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20 °C.

Verbindungen aus den Tabellen 1 und 2 zeigten gegen den Phytophthora-Pilz eine gute Schutzwirkung. So reduzierten z.B. die Verbindungen 1.1, 1.2, 1.11, 1.12, 1.24, 1.25, 1.77, 1.85, 1.89, 1.91 und 1.93 den Pilzbefall auf 0 bis 20 %. Unbehandelte jedoch infizierte Kontrollpflanzen wiesen dagegen einen 100 %igen Phytophthora-Befall auf.

Beispiel 3.4: Wirkung gegen Plasmopara viticola auf Reben

a) Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20 °C wird der Pilzbefall beurteilt.

b) Im 4-5 Blattstadium werden Rebensämlinge mit einer Sporangiensuspension des Pilzes infiziert. Nach einer Inkubation während 24 Stunden in einer Feuchtkammer bei 95-100 % relativer Luftfeuchtigkeit und 20 °C werden die infizierten Pflanzen getrocknet und mit einer aus Spritzpulver des Wirkstoffes hergestellten Spritzbrühe (0,06 % Aktivsubstanz) besprüht. Nach dem Antrocknen des Spritzbelages werden die behandelten Pflanzen wieder in die Feuchtkammer gebracht. Die Beurteilung des Pilzbefalls erfolgt 6 Tage nach der Infektion.

Verbindungen aus den Tabellen 1 und 2 zeigten gegen Plasmopara viticola eine gute Schutzwirkung (Befall 0-20 %), so z.B. die Verbindungen 1.4, 1.20 und 1.67. Unbehandelte jedoch infizierte Kontrollpflanzen wiesen dagegen einen 100%igen Plasmopara-Befall auf.

Beispiel 3.5: Wirkung gegen Pyricularia oryzae auf Reispflanzen

a) Reispflanzen werden nach 2-wöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24 °C wird der Pilzbefall beurteilt. b) Zu 2-wöchigen Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95 - 100 % relativer Luftfeuchtigkeit und ca. 24 °C wird der Pilzbefall beurteilt.

Reispflanzen, die mit einer Spritzbrühe behandelt wurden, die als Aktivsubstanz eine Verbindung aus den Tabellen 1 und 2 enthielt, zeigten im Vergleich zu unbehandelten Kontrollpflanzen (100 % Befall) nur geringen Pilzbefall. So reduzierten z.B. im Test (a) die Verbindungen 1.1, 1.2, 1.4, 1.6, 1.18, 1.19, 1.20, 1.65, 1.67, 1.77, 1.93, 1.98 und im Test b) die Verbindungen 1.4, 1.6, 1.8, 1.9, 1.19, 1.20, 1.62, 1.65, 1.67, 1.77, 2.3, 2.4 und 2.5 den Befall auf 0 bis 20 %.

Beispiel 3.6: Beizung gegen Pyricularia oryzae auf Reispflanzen

100 kg Trockenreis ("Upland-Rice") werden ca. 1 Stunde lang in einem Mischgefäss mit Spritzpulver (Formulierung WP-25), das 200 g A.S. enthält, behandelt bis die Oberfläche der Reiskörner mit dem formulierten Wirkstoff gleichmässig überzogen ist.

Das so präparierte Reis-Saatgut wird auf 10 $m^2$ grosse Parzellen ausgesät, deren Randstreifen mit Reispflanzen besetzt sind, die einen Befall von Pyricularia oryzae (= leaf blast) aufweisen. Nach dem Auflaufen der Reissaat findet die erste Befallauswertung statt sobald die Reispflanzen Blattflecken zeigen. Danach werden weitere Auswertungen in einwöchigen Intervallen durchgeführt.

Verbindungen aus den Tabellen 1 und 2 zeigten in dem Test gute Wirkung. So begrenzte die Verbindung 1.12 oder 1.20 den Pyricularia-Befall der Reispflanzen auf 0 bis 20 %. Kontrollpflanzen aus unbehandeltem Saatgut wiesen dagegen einen Krankheitsbefall von 100 % auf.

Beispiel 3.7: Wirkung gegen Xanthomonas oryzae auf Reis (Oryza sativa)

a) Reispflanzen der Sorte "Calora" oder "S6" werden nach 3-wöchiger Anzucht im Gewächshaus mit der Prüfsubstanz in Form einer Spritzbrühe (0,02 % Aktivsubstanz) besprüht. Hach eintägigem Antrocken dieses Spritzbelags werden die Pflanzen in einem Klimaraum bei 24 °C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der residual Wirksamkeit der Prüfsubstanz.

b) Reispflanzen der Sorte "Calora" oder "S6" werden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung werden die Pflanzen in einem Klimaraum bei 24 °C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der systemischen Wirksamkeit der Prüfsubstanz.

Verbindungen aus den Tabellen 1 und 2 zeigten eine gute Schutzwirkung gegen Xanthomonas oryzae. So reduzierten z.B. im Test (a) die Verbindungen 1.1, 1.6, 1.24 und 1.25 sowie im Test (b) die Verbindungen 1.1, 1.6, 1.18, 1.20, 1.24, 1.25 und 1.62 den Bakterienbefall auf 0 bis 20 %. Unbehandelte jedoch infizierte Kontrollpflanzen wiesen dagegen einen Krankheitsbefall von 100 % auf.

Beispiel 3.8: Wirkung gegen Xanthomonas vesicatoria auf Paprika (Capsicum annuum)

a) Paprikapflanzen der Sorte "California Wonder" werden nach 3-wöchiger Anzucht im Gewächshaus mit der Prüfsubstanz in Form einer Spritzbrühe (0,02 % Aktivsubstanz) besprüht. Nach eintägigem Antrocknen dieses Spritzbelages werden die Pflanzen in einem Klimaraum bei 26 °C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation bilden sich bei Befall auf den Blättern runde, anfangs wässrige, später nekrotische, aufgehellte Flecken. Das Ausmass dieser Flecken dient zur Beurteilung der residualen Wirksamkeit der Prüfsubstanz.

b) Paprikapflanzen der Sorte "California Wonder" werden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung werden die Pflanzen in einem Klimaraum bei 26 °C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation bilden sich bei Befall auf den Blättern runde, anfangs wässrige, später nekrotische, aufgehellte Flecken. Das Ausmass dieser Flecken dient zur Beurteilung der systemischen Wirksamkeit der Prüfsubstanz.

Verbindungen aus den Tabellen 1 und 2 zeigten eine gute Schutzwirkung gegen Xanthomonas vesicatoria. So reduzierten z.B. im Test (a) die Verbindung 1.24 und im Test (b) die Verbindungen 1.1, 1.20, 1.24, 1.25 und 1.62 den Bakterienbefall auf 0 bis 20 %. Unbehandelte jedoch infizierte Kontrollpflanzen wiesen dagegen einen Krankheitsbefall von 100 % auf.

Beispiel 3.9: Wirkung gegen Pseudomonas tomato an Tomatenpflanzen

a) Tomatenpflanzen werden nach 3-wöchiger Anzucht mit einer aus Spritzpulver des Wirkstoffes hergestellten Spritzbrühe durch Blattapplikation behandelt (Konzentration: 200 ppm). Nach 3,5 Wochen werden die Pflanzen mit einer Bakteriensuspension ($10^8$ Bakterien/ml) inokuliert und für 6 Tage bei hoher Luftfeuchtigkeit und bei einer Temperatur von 25 °C inkubiert. Die Beurteilung der Schutzwirkung erfolgt aufgrund des Bakterienbefalls 7-8 Tage nach Inokulation.

Unbehandelte aber infizierte Kontrollpflanzen weisen in diesem Test einen Befall von 100 % auf.

Verbindungen aus den Tabellen 1 und 2 zeigten eine gute Schutzwirkung gegen Pseudomonas tomato. So blieben Pflanzen, die z.B. mit der Verbindung 1.20, 1.24 oder 1.25 behandelt wurden, weitgehend frei von Pseudomonas (Befall: 20 bis O %).

b) Tomatenpflanzen werden nach 3-wöchiger Anzucht mit einer aus Spritzpulver des Wirkstoffes hergestellten Spritzbrühe durch Bodenapplikation behandelt (Konzentration: 60 ppm bezogen auf das Bodenvolumen). Nach 3,5 Wochen werden die Pflanzen mit Bakteriensuspension ($10^8$ Bakterien/ml) inokuliert und für 6 Tage bei hoher Luftfeuchtigkeit und bei einer Temperatur von 25 °C inkubiert.

Die Beurteilung der Schutzwirkung erfolgt aufgrund des Bakterienbefalls 7-8 Tage nach Inokulation.

Unbehandelte aber infizierte Kontrollpflanzen weisen in diesem Test einen Befall von 100 % auf.

Verbindungen aus den Tabellen 1 und 2 bewirken eine gute Wirkung gegen Pseudomonas tomato. So blieben Pflanzen, die z.B. mit der Verbindung 1.1, 1.20, 1.24 oder 1.25 behandelt wurden, fast völlig frei von Pseudomonas (Befall: 20 bis 0 %).

Beispiel 3.10: Wirkung gegen Erysiphe graminis auf Gerste

a) Ca. 8 cm hohe Gerstenpflanzen werden mit einer aus Spritzpulver des Wirkstoffes hergestellten Spritzbrühe (O,O2 % Aktivsubstanz) besprüht. Nach 3 - 4 Stunden werden die behandelten Pflanzen mit Konidien des Pilzes bestäubt. Die infizierten Gerstenpflanzen werden in einem Gewächshaus bei ca. 22 °C aufgestellt und der Pilzbefall nach 10 Tagen beurteilt.

b) Zu ca. 8 cm hohen Gerstenpflanzen wird eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit Konidien des Pilzes bestäubt. Die infizierten Gerstenpflanzen werden in einem Gewächshaus bei ca. 22 °C aufgestellt und der Pilzbefall nach 10 Tagen beurteilt.

Verbindungen aus den Tabellen 1 und 2, z.B. die Verbindung 1.20 reduzierte den Pilzbefall auf weniger als 20 %, während unbehandelte aber infizierte Kontrollpflanzen zu 100 % befallen waren.

## Patentansprüche

## Patentansprüche für folgende Vertragsstaaten : AT, BE, CH, DE, FR, GB, GR, IT, LI, LU, NL, SE

**1.** Verbindungen der Formel I

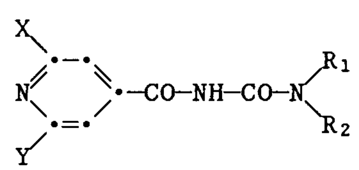

(I)

in welcher bedeuten:

X Wasserstoff oder Halogen;

Y Halogen;

$R_1$, $R_2$ unabhängig voneinander Wasserstoff, $C_1$-$C_6$-Alkyl, mit Halogen, Cyano, $C_1$-$C_4$-Alkoxy oder COO-$C_1$-$C_3$-Alkyl substituiertes $C_1$-$C_6$-Alkyl, $C_3$-$C_5$-Alkenyl, mit Halogen substituiertes $C_3$-$C_5$-Alkenyl, $C_3$-$C_5$-Alkinyl, mit Halogen substituiertes $C_3$-$C_5$-Alkinyl, $C_3$-$C_7$-Cycloalkyl, mit $C_1$-$C_2$-Alkyl, Halogen, Cyano oder COO-$C_1$-$C_3$-Alkyl substituiertes $C_3$-$C_7$-Cycloalkyl, Phenyl, Benzyl, mit $C_1$-$C_3$-Alkyl, $C_1$-$C_3$-Alkoxy oder Halogen substituiertes Phenyl oder Benzyl; und $R_1$ weiterhin auch den Rest Q oder mit $C_1$-$C_3$-Alkyl, $C_3$-$C_7$-Cycloalkyl, $C_1$-$C_3$-Alkoxy oder Halogen substituiertes Q darstellen kann, wobei Q über ein $C_1$-$C_2$-Alkyl an das N-Atom gebunden sein kann; oder worin $R_1$ und $R_2$ zusammen mit dem benachbarten N-Atom einen 3- bis 7-gliedrigen Heterocyclus bilden, der zusätzlich 1 oder

2 weitere Heteroatome, wie O, N oder S, und eine Carbonylgruppe enthalten und mit $C_1$-$C_3$-Alkyl, Halogen oder COO-$C_1$-$C_3$-Alkyl substituiert sein kann;

Q Furan-2-yl, Thiophen-2-yl, Isoxazol-3-yl, Isoxazol-5-yl, Thiazol-2-yl, 1,2,4-Thiadiazol-3-yl, 1,3,4-Thiadiazol-2-yl, 2-, 3- oder 4-Pyridylreste, mit $C_1$-$C_3$-Alkyl, $C_1$-$C_3$-Alkoxy, $C_1$-$C_3$-Alkylthio oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy, $C_1$-$C_3$-Alkylthio, Halogen oder Cyclopropyl substituierte 2-, 4- oder 5-Pyrimidylreste, mit Ausnahme der Verbindung 2,6-Dichloro-N-(((4-chlorophenyl)amino)carbonyl)-4-pyridincarboxamid.

**2.** Verbindungen der Formel I

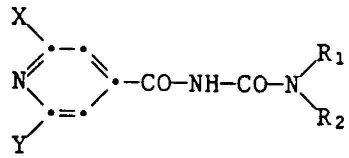

(I)

in welcher bedeuten:

X Wasserstoff oder Halogen;

Y Halogen;

$R_1$, $R_2$ unabhängig voneinander Wasserstoff, $C_1$-$C_6$-Alkyl, mit Halogen, Cyano oder COO-$C_1$-$C_3$-Alkyl substituiertes $C_1$-$C_6$-Alkyl, $C_3$-$C_5$-Alkenyl, mit Halogen substituiertes $C_3$-$C_5$-Alkenyl, $C_3$-$C_5$-Alkinyl, mit Halogen substituiertes $C_3$-$C_5$-Alkinyl, $C_3$-$C_7$-Cycloalkyl, mit $C_1$-$C_2$-Alkyl, Halogen, Cyano oder COO-$C_1$-$C_3$-Alkyl substituiertes $C_3$-$C_7$-Cycloalkyl, Benzyl, mit $C_1$-$C_3$-Alkyl, $C_1$-$C_3$-Alkoxy oder Halogen substituiertes Benzyl; und $R_1$ weiterhin auch den Rest Q oder mit $C_1$-$C_3$-Alkyl, $C_3$-$C_7$-Cycloalkyl, $C_1$-$C_3$-Alkoxy oder Halogen substituiertes Q darstellen kann, wobei Q über ein $C_1$-$C_2$-Alkyl an das N-Atom gebunden sein kann; oder worin $R_1$ und $R_2$ zusammen mit dem benachbarten N-Atom einen 3- bis 7-gliedrigen Heterocyclus bilden, der zusätzlich 1 oder 2 weitere Heteroatome, wie O, N oder S, und eine Carbonylgruppe enthalten und mit $C_1$-$C_3$-Alkyl, Halogen oder COO-$C_1$-$C_3$-Alkyl substituiert sein kann;

Q Furan-2-yl, Thiophen-2-yl, Isoxazol-3-yl, Isoxazol-5-yl, Thiazol-2-yl, 1,2,4-Thiadiazol-3-yl, 1,3,4-Thiadiazol-2-yl, 2-, 3- oder 4-Pyridylreste, mit $C_1$-$C_3$-Alkyl, $C_1$-$C_3$-Alkoxy, $C_1$-$C_3$-Alkylthio oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy, $C_1$-$C_3$-Alkylthio, Halogen oder Cyclopropyl substituierte 2-, 4- oder 5-Pyrimidylreste.

**3.** Verbindungen gemäss Anspruch 2, worin bedeuten:

X Wasserstoff, Fluor, Chlor oder Brom;

Y Fluor, Chlor oder Brom;

$R_1$ $C_1$-$C_6$-Alkyl, mit Fluor, Chlor, Cyano oder COO-$C_1$-$C_3$-Alkyl substituiertes $C_1$-$C_6$-Alkyl, $C_3$-$C_5$-Alkenyl, mit Chlor substituiertes Alkenyl, $C_3$-$C_5$-Alkinyl, mit Chlor substituiertes $C_3$-$C_5$-Alkinyl, $C_3$-$C_7$-Cycloalkyl, mit COO-$C_1$-$C_3$-Alkoxy substituiertes $C_3$-$C_7$-Cycloalkyl, Benzyl, mit $C_1$-$C_3$-Alkoxy oder Halogen substituiertes Benzyl oder Q;

$R_2$ Wasserstoff, $C_1$-$C_6$-Alkyl oder mit Fluor, Chlor, Cyano oder COO-$C_1$-$C_3$-Alkyl substituiertes $C_1$-$C_6$-Alkyl;

$R_1$ und $R_2$ bilden zusammen mit dem benachbarten N-Atom einen 4- bis 7-gliedrigen Heterocyclus, der zusätzlich ein weiteres N-Atom oder ein O-Atom enthalten und mit Methyl oder COO-$C_1$-$C_3$-Alkyl substituiert sein kann;

Q Furan-2-yl, Thiophen-2-yl, Isoxazol-3-yl oder Isoxazol-5-yl, 2-, 3- oder 4-Pyridylreste, mit $C_1$-$C_3$-Alkyl, $C_1$-$C_3$-Alkoxy oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy oder Halogen substituierte 2-, 4- oder 5-Pyrimidylreste.

**4.** Verbindungen gemäss Anspruch 2, worin bedeuten:

X Wasserstoff, Fluor, Chlor oder Brom;

Y Fluor, Chlor oder Brom;

| | |
|---|---|
| $R_1$ | $C_1$-$C_6$-Alkyl, mit Halogen substituiertes $C_1$-$C_6$-Alkyl oder Q; |
| $R_2$ | Wasserstoff, $C_1$-$C_6$-Alkyl oder mit Fluor, Chlor, Cyano oder COO-$C_1$-$C_3$-Alkyl substituiertes $C_1$-$C_6$-Alkyl; |
| $R_1$ und $R_2$ | bilden zusammen mit dem benachbarten N-Atom einen 4- bis 7-gliedrigen Heterocyclus, der zusätzlich ein N- oder ein O-Atom enthalten und mit Halogen substituiert sein kann; |
| Q | Thiazol-2-yl, 1,2,4-Thiazol-3-yl, 1,3,4-Thiadiazol-2-yl, 2-, 3- oder 4-Pyridylreste oder mit $C_1$-$C_3$-Alkyl, $C_1$-$C_3$-Alkoxy oder Halogen substituierte 2-, 3- oder 4-Pyridylreste. |

**5.** Verbindungen gemäss Anspruch 3, worin bedeuten:

| | |
|---|---|
| X | Wasserstoff, Chlor oder Brom; |
| Y | Chlor oder Brom; |
| $R_1$ | $C_1$-$C_4$-Alkyl, mit Chlor oder COO-$C_1$-$C_3$-Alkyl substituiertes $C_1$-$C_4$-Alkyl, $C_3$-$C_5$-Alkenyl, $C_3$-$C_5$-Alkinyl, Cyclopropyl, mit $COOCH_3$ substituiertes Cyclopropyl, Benzyl, mit Methoxy oder Chlor substituiertes Benzyl oder Q; |
| $R_2$ | Wasserstoff, $C_1$-$C_4$-Alkyl, mit Chlor oder COO-$C_1$-$C_3$-Alkyl substituiertes $C_1$-$C_4$-Alkyl; |
| $R_1$ und $R_2$ | bilden zusammen mit dem benachbarten N-Atom einen 5- oder 6-gliedrigen Heterocyclus, der zusätzlich ein weiteres N- oder ein O-Atom enthalten und mit Methyl oder COO-$C_1$-$C_3$-Alkyl substituiert sein kann; |
| Q | Furan-2-yl, Thiophen-2-yl, Isoxazol-3-yl, Isoxazol-5-yl, 2-, 3- oder 4-Pyridylreste, mit $C_1$-$C_3$-Alkyl, $C_1$-$C_3$-Alkoxy, Chlor oder Brom substituierte 2-, 3- oder 4-Pyridylreste; 2-, 4- oder 5-Pyrimidylreste oder mit $C_1$-$C_3$-Alkyl, $C_1$-$C_3$-Alkoxy, Chlor oder Brom substituierte 2-, 4- oder 5-Pyrimidylreste. |

**6.** Verbindungen gemäss Anspruch 4, worin bedeuten:

| | |
|---|---|
| X | Wasserstoff, Chlor oder Brom; |
| Y | Chlor oder Brom; |
| $R_1$ | $C_1$-$C_4$-Alkyl, mit Fluor oder Chlor substituiertes $C_1$-$C_4$-Alkyl oder Q; |
| $R_2$ | Wasserstoff, $C_1$-$C_4$-Alkyl, mit Chlor oder COO-$C_1$-$C_3$-Alkyl substituiertes $C_1$-$C_4$-Alkyl; |
| $R_1$ und $R_2$ | bilden zusammen mit dem benachbarten N-Atom einen 5- oder 6-gliedrigen Heterocyclus, der zusätzlich ein weiteres N-Atom oder ein O-Atom enthalten und mit Chlor oder Brom substituiert sein kann; |
| Q | Thiazol-2-yl, 1,2,4-Thiadiazol-3-yl, 1,3,4-Thiadiazol-2-yl, 2-, 3- oder 4-Pyridylreste oder mit Methyl, Methoxy, Fluor, Chlor oder Brom substituierte 2-, 3- oder 4-Pyridylreste. |

**7.** N-(2,6-Dichlorisonicotinoyl)-carbamoyl-piperidin, gemäß Anspruch 1.

**8.** N,N-Dimethyl-N′-(2,6-dichlorisonicotinoyl)-harnstoff, gemäß Anspruch 1.

**9.** Verfahren zur Herstellung von Verbindungen der Formel I in Anspruch 1, dadurch gekennzeichnet, dass man umsetzt:
ein Isonicotinsäureamid der Formel II

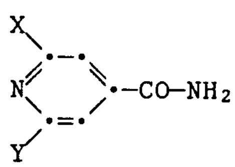

(II)

mit

a) einem Carbamoylhalogenid der Formel IIIa

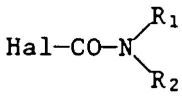 (IIIa)

oder
b) einem Carbamoylazolid der Formel IIIb

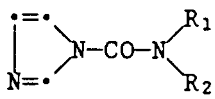 (IIIb)

oder zur Herstellung der Verbindungen der Formel I, in welchen $R_1$ oder $R_2$ Wasserstoff darstellt,
c) mit einem Isocyanat der Formel IIIc

O = C = N-A (IIIc)

in Gegenwart einer Base in aprotischen Lösungsmitteln, wobei in den vorstehend genannten Verbindungen Hal Halogen, bevorzugt Chlor, darstellt, A die Bedeutung von $R_1$ bzw. $R_2$ hat und dieser Rest sowie X und Y die unter Formel I angegebenen Bedeutungen haben.

**10.** Verfahren zur Herstellung von Verbindungen der Formel I in Anspruch 1, dadurch gekennzeichnet, dass man reagieren lässt:
eine Verbindung der Formel II

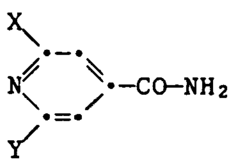 (II)

a) mit $(COCl)_2$ oder $CO(Cl)_2$ zu einem Isocyanat der Formel IV

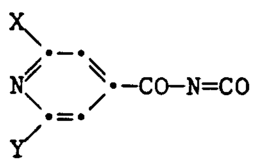 (IV),

welches anschliessend
b) mit einem Amin der Formel V

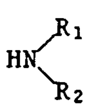 (V)

umgesetzt wird, wobei die Reaktionsschritte in inerten Lösungsmitteln ablaufen.

**11.** Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung gemäss Anspruch 1 enthält.

**12.** Mittel gemäss Anspruch 11, dadurch gekennzeichnet, dass es als aktive Komponente mindestens eine Verbindung gemäss den Ansprüchen 2 bis 6 enthält.

**13.** Mittel gemäss Anspruch 11, dadurch gekennzeichnet, dass es als aktive Komponente die Verbindung der Formel I gemäss Anspruch 7 enthält.

**14.** Mittel gemäss Anspruch 11, dadurch gekennzeichnet, dass es als aktive Komponente die Verbindung der Formel I gemäss Anspruch 8 enthält.

**15.** Verwendung von Verbindungen gemäss Anspruch 1 zum Schutz von Pflanzen gegen den Befall durch phytopathogene Mikroorganismen.

**16.** Verwendung von Verbindungen gemäss Anspruch 2 zum Schutz von Pflanzen gegen den Befall durch phytopathogene Mikroorganismen.

**Patentansprüche für folgenden Vertragsstaat : ES**

**1.** Verfahren zur Herstellung von Verbindungen der Formel I

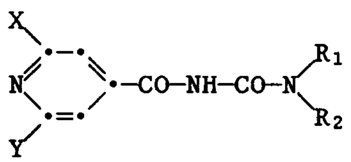

(I)

in welcher bedeuten:

X Wasserstoff oder Halogen;

Y Halogen;

$R_1$, $R_2$ unabhängig voneinander Wasserstoff, $C_1$-$C_6$-Alkyl, mit Halogen, Cyano, $C_1$-$C_4$-Alkoxy oder COO-$C_1$-$C_3$-Alkyl substituiertes $C_1$-$C_6$-Alkyl, $C_3$-$C_5$-Alkenyl, mit Halogen substituiertes $C_3$-$C_5$-Alkenyl, $C_3$-$C_5$-Alkinyl, mit Halogen substituiertes $C_3$-$C_5$-Alkinyl, $C_3$-$C_7$-Cycloalkyl, mit $C_1$-$C_2$-Alkyl, Halogen, Cyano oder COO-$C_1$-$C_3$-Alkyl substituiertes $C_3$-$C_7$-Cycloalkyl, Phenyl, Benzyl, mit $C_1$-$C_3$-Alkyl, $C_1$-$C_3$-Alkoxy oder Halogen substituiertes Phenyl oder Benzyl; und $R_1$ weiterhin auch den Rest Q oder mit $C_1$-$C_3$-Alkyl, $C_3$-$C_7$-Cycloalkyl, $C_1$-$C_3$-Alkoxy oder Halogen substituiertes Q darstellen kann, wobei Q über ein $C_1$-$C_2$-Alkyl an das N-Atom gebunden sein kann; oder worin $R_1$ und $R_2$ zusammen mit dem benachbarten N-Atom einen 3- bis 7-gliedrigen Heterocyclus bilden, der zusätzlich 1 oder 2 weitere Heteroatome, wie O, N oder S, und eine Carbonylgruppe enthalten und mit $C_1$-$C_3$-Alkyl, Halogen oder COO-$C_1$-$C_3$-Alkyl substituiert sein kann;

Q Furan-2-yl, Thiophen-2-yl, Isoxazol-3-yl, Isoxazol-5-yl, Thiazol-2-yl, 1,2,4-Thiadiazol-3-yl, 1,3,4-Thiadiazol-2-yl, 2-, 3- oder 4-Pyridylreste, mit $C_1$-$C_3$-Alkyl, $C_1$-$C_3$-Alkoxy, $C_1$-$C_3$-Alkylthio oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy, $C_1$-$C_3$-Alkylthio, Halogen oder Cyclopropyl substituierte 2-, 4- oder 5-Pyrimidylreste, mit Ausnahme der Verbindung 2,6-Dichloro-N-(((4-chlorophenyl)amino)-carbonyl)-4-pyridincarboxamid, dadurch gekennzeichnet, dass man umsetzt:

ein Isonicotinsäureamid der Formel II

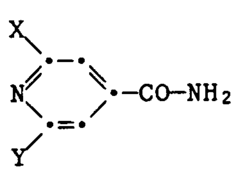

(II)

mit

a) einem Carbamoylhalogenid der Formel IIIa

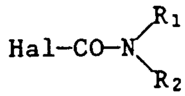

(IIIa)

oder

b) einem Carbamoylazolid der Formel IIIb

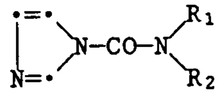

(IIIb)

oder zur Herstellung der Verbindungen der Formel I, in welchen $R_1$ oder $R_2$ Wasserstoff darstellt,

c) mit einem Isocyanat der Formel IIIc

O = C = N-A (IIIc)

in Gegenwart einer Base in aprotischen Lösungsmitteln, wobei in den vorstehend genannten Verbindungen Hal Halogen, bevorzugt Chlor, darstellt, A die Bedeutung von $R_1$ bzw. $R_2$ hat und dieser Rest sowie X und Y die unter Formel I angegebenen Bedeutungen haben.

**2.** Verfahren zur Herstellung von Verbindungen der Formel I

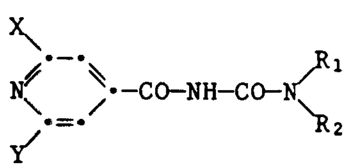

(I)

in welcher bedeuten:

X Wasserstoff oder Halogen;

Y Halogen;

$R_1$, $R_2$ unabhängig voneinander Wasserstoff, $C_1$-$C_6$-Alkyl, mit Halogen, Cyano, $C_1$-$C_4$-Akoxy oder COO-$C_1$-$C_3$-Alkyl substituiertes $C_1$-$C_6$-Alkyl, $C_3$-$C_5$-Alkenyl, mit Halogen substituiertes $C_3$-$C_5$-Alkenyl, $C_3$-$C_5$-Alkinyl, mit Halogen substituiertes $C_3$-$C_5$-Alkinyl, $C_3$-$C_7$-Cycloalkyl, mit $C_1$-$C_2$-Alkyl, Halogen, Cyano oder COO-$C_1$-$C_3$-Alkyl substituiertes $C_3$-$C_7$-Cycloalkyl, Phenyl, Benzyl, mit $C_1$-$C_3$-Alkyl, $C_1$-$C_3$-Alkoxy oder Halogen substituiertes Phenyl oder Benzyl; und $R_1$ weiterhin auch den Rest Q oder mit $C_1$-$C_3$-Alkyl, $C_3$-$C_7$-Cycloalkyl, $C_1$-$C_3$-Alkoxy oder Halogen substituiertes Q darstellen kann, wobei Q über ein $C_1$-$C_2$-Alkyl an das N-Atom gebunden sein kann; oder worin $R_1$ und $R_2$ zusammen mit dem benachbarten N-Atom einen 3- bis 7-gliedrigen Heterocyclus bilden, der zusätzlich 1 oder 2 weitere Heteroatome, wie O, N oder S, und eine Carbonylgruppe enthalten und mit $C_1$-$C_3$-Alkyl, Halogen oder COO-$C_1$-$C_3$-Alkyl substituiert sein kann;

Q Furan-2-yl, Thiophen-2-yl, Isoxazol-3-yl, Isoxazol-5-yl, Thiazol-2-yl, 1,2,4-Thiadiazol-3-yl, 1,3,4-Thiadiazol-2-yl, 2-, 3- oder 4-Pyridylreste, mit $C_1$-$C_3$-Alkyl, $C_1$-$C_3$-Alkoxy, $C_1$-$C_3$-Alkylthio oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy, $C_1$-$C_3$-Alkylthio, Halogen oder Cyclopropyl substituierte 2-, 4- oder 5-Pyrimidylreste, dadurch gekennzeichnet, dass man reagieren lässt:

eine Verbindung der Formel II

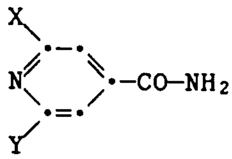

(II)

a) mit $(COCl)_2$ oder $CO(Cl)_2$ zu einem Isocyanat der Formel IV

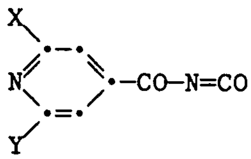

(IV),

welches anschliessend
b) mit einem Amin der Formel V

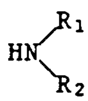

(V)

umgesetzt wird, wobei die Reaktionsschritte in inerten Lösungsmitteln ablaufen und X, Y, $R_1$ und $R_2$ die unter Formel I angegebenen Bedeutungen haben.

**3.** Verfahren gemäss Anspruch 1 oder 2, dadurch gekennzeichnet, dass Verbindungen der Formel I hergestellt werden, worin bedeuten:

X Wasserstoff oder Halogen;

Y Halogen;

$R_1$, $R_2$ unabhängig voneinander Wasserstoff, $C_1$-$C_6$-Alkyl, mit Halogen, Cyano oder COO-$C_1$-$C_3$-Alkyl substituiertes $C_1$-$C_6$-Alkyl, $C_3$-$C_5$-Alkenyl, mit Halogen substituiertes $C_3$-$C_5$-Alkenyl, $C_3$-$C_5$-Alkinyl, mit Halogen substituiertes $C_3$-$C_5$-Alkinyl, $C_3$-$C_7$-Cycloalkyl, mit $C_1$-$C_2$-Alkyl, Halogen, Cyano oder COO-$C_1$-$C_3$-Alkyl substituiertes $C_3$-$C_7$-Cycloalkyl, Benzyl, mit $C_1$-$C_3$-Alkyl, $C_1$-$C_3$-Alkoxy oder Halogen substituiertes Benzyl; und $R_1$ weiterhin auch den Rest Q oder mit $C_1$-$C_3$-Alkyl, $C_3$-$C_7$-Cycloalkyl, $C_1$-$C_3$-Alkoxy oder Halogen substituiertes Q darstellen kann, wobei Q über ein $C_1$-$C_2$-Alkyl an das N-Atom gebunden sein kann; oder worin $R_1$ und $R_2$ zusammen mit dem benachbarten N-Atom einen 3- bis 7-gliedrigen Heterocyclus bilden, der zusätzlich 1 oder 2 weitere Heteroatome, wie O, N oder S, und eine Carbonylgruppe enthalten und mit $C_1$-$C_3$-Alkyl, Halogen oder COO-$C_1$-$C_3$-Alkyl substituiert sein kann;

Q Furan-2-yl, Thiophen-2-yl, Isoxazol-3-yl, Isoxazol-5-yl, Thiazol-2-yl, 1,2,4-Thiadiazol-3-yl, 1,3,4-Thiadiazol-2-yl, 2-, 3- oder 4-Pyridylreste, mit $C_1$-$C_3$-Alkyl, $C_1$-$C_3$-Alkoxy, $C_1$-$C_3$-Alkylthio oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy, $C_1$-$C_3$-Alkylthio, Halogen oder Cyclopropyl substituierte 2-, 4- oder 5-Pyrimidylreste.

**4.** Verfahren gemäss Anspruch 1 oder 2, dadurch gekennzeichnet, dass Verbindungen der Formel I hergestellt werden, worin bedeuten:

X Wasserstoff, Fluor, Chlor oder Brom;

Y Fluor, Chlor oder Brom;

$R_1$ $C_1$-$C_6$-Alkyl, mit Fluor, Chlor, Cyano oder COO-$C_1$-$C_3$-Alkyl substituiertes $C_1$-$C_6$-Alkyl, $C_3$-$C_5$-Alkenyl, mit Chlor substituiertes Alkenyl, $C_3$-$C_5$-Alkinyl, mit Chlor substituiertes $C_3$-$C_5$-Alkinyl, $C_3$-$C_7$-Cycloalkyl, mit COO-$C_1$-$C_3$-Alkoxy substituiertes $C_3$-$C_7$-Cycloalkyl, Benzyl, mit $C_1$-$C_3$-Alkoxy oder Halogen substituiertes Benzyl oder Q;

$R_2$ Wasserstoff, $C_1$-$C_6$-Alkyl oder mit Fluor, Chlor, Cyano oder COO-$C_1$-$C_3$-Alkyl substituiertes $C_1$-$C_6$-Alkyl;

| | |
|---|---|
| $R_1$ und $R_2$ | bilden zusammen mit dem benachbarten N-Atom einen 4- bis 7-gliedrigen Heterocyclus, der zusätzlich ein weiteres N-Atom oder ein O-Atom enthalten und mit Methyl oder COO-$C_1$-$C_3$-Alkyl substituiert sein kann; |
| Q | Furan-2-yl, Thiophen-2-yl, Isoxazol-3-yl oder Isoxazol-5-yl, 2-, 3- oder 4-Pyridylreste, mit $C_1$-$C_3$-Alkyl, $C_1$-$C_3$-Alkoxy oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy oder Halogen substituierte 2-, 4- oder 5-Pyrimidylreste. |

**5.** Verfahren gemäss Anspruch 1 oder 2, dadurch gekennzeichnet, dass Verbindungen der Formel I hergestellt werden, worin bedeuten:

| | |
|---|---|
| X | Wasserstoff, Fluor, Chlor oder Brom; |
| Y | Fluor, Chlor oder Brom; |
| $R_1$ | $C_1$-$C_6$-Alkyl, mit Halogen substituiertes $C_1$-$C_6$-Alkyl oder Q; |
| $R_2$ | Wasserstoff, $C_1$-$C_6$-Alkyl oder mit Fluor, Chlor, Cyano oder COO-$C_1$-$C_3$-Alkyl substituiertes $C_1$-$C_6$-Alkyl; |
| $R_1$ und $R_2$ | bilden zusammen mit dem benachbarten N-Atom einen 4- bis 7-gliedrigen Heterocyclus, der zusätzlich ein N- oder ein O-Atom enthalten und mit Halogen substituiert sein kann; |
| Q | Thiazol-2-yl, 1,2,4-Thiazol-3-yl, 1,3,4-Thiadiazol-2-yl, 2-, 3- oder 4-Pyridylreste oder mit $C_1$-$C_3$-Alkyl, $C_1$-$C_3$-Alkoxy oder Halogen substituierte 2-, 3- oder 4-Pyridylreste. |

**6.** Verfahren gemäss Anspruch 1 oder 2, dadurch gekennzeichnet, dass Verbindungen der Formel I hergestellt werden, worin bedeuten:

| | |
|---|---|
| X | Wasserstoff, Chlor oder Brom; |
| Y | Chlor oder Brom; |
| $R_1$ | $C_1$-$C_4$-Alkyl, mit Chlor oder COO-$C_1$-$C_3$-Alkyl substituiertes $C_1$-$C_4$-Alkyl, $C_3$-$C_5$-Alkenyl, $C_3$-$C_5$-Alkinyl, Cyclopropyl, mit $COOCH_3$ substituiertes Cyclopropyl, Benzyl, mit Methoxy oder Chlor substituiertes Benzyl oder Q; |
| $R_2$ | Wasserstoff, $C_1$-$C_4$-Alkyl, mit Chlor oder COO-$C_1$-$C_3$-Alkyl substituiertes $C_1$-$C_4$-Alkyl; |
| $R_1$ und $R_2$ | bilden zusammen mit dem benachbarten N-Atom einen 5- oder 6-gliedrigen Heterocyclus, der zusätzlich ein weiteres N- oder ein O-Atom enthalten und mit Methyl oder COO-$C_1$-$C_3$-Alkyl substituiert sein kann; |
| Q | Furan-2-yl, Thiophen-2-yl, Isoxazol-3-yl, Isoxazol-5-yl, 2-, 3- oder 4-Pyridylreste, mit $C_1$-$C_3$-Alkyl, $C_1$-$C_3$-Alkoxy, Chlor oder Brom substituierte 2-, 3- oder 4-Pyridylreste; 2-, 4- oder 5-Pyrimidylreste oder mit $C_1$-$C_3$-Alkyl, $C_1$-$C_3$-Alkoxy, Chlor oder Brom substituierte 2-, 4- oder 5-Pyrimidylreste. |

**7.** Verfahren gemäss Anspruch 1 oder 2, dadurch gekennzeichnet, dass Verbindungen der Formel I hergestellt werden, worin bedeuten:

| | |
|---|---|
| X | Wasserstoff, Chlor oder Brom; |
| Y | Chlor oder Brom; |
| $R_1$ | $C_1$-$C_4$-Alkyl, mit Fluor oder Chlor substituiertes $C_1$-$C_4$-Alkyl oder Q; |
| $R_2$ | Wasserstoff, $C_1$-$C_4$-Alkyl, mit Chlor oder COO-$C_1$-$C_3$-Alkyl substituiertes $C_1$-$C_4$-Alkyl; |
| $R_1$ und $R_2$ | bilden zusammen mit dem benachbarten N-Atom einen 5- oder 6-gliedrigen Heterocyclus, der zusätzlich ein weiteres N-Atom oder ein O-Atom enthalten und mit Chlor oder Brom substituiert sein kann; |
| Q | Thiazol-2-yl, 1,2,4-Thiadiazol-3-yl, 1,3,4-Thiadiazol-2-yl, 2-, 3- oder 4-Pyridylreste oder mit Methyl, Methoxy, Fluor, Chlor oder Brom substituierte 2-, 3- oder 4-Pyridylreste. |

**8.** Verfahren gemäss Anspruch 1 oder 2, dadurch gekennzeichnet, dass N-(2,6-Dichlorisonicotinoyl)-carbamoyl-piperidin hergestellt wird.

**9.** Verfahren gemäss Anspruch 1 oder 2, dadurch gekennzeichnet, dass N,N-Dimethyl-N'-(2,6-dichlorisonicotinoyl)-harnstoff hergestellt wird.

**10.** Mittel zum Schutz von Pflanzen gegen den Befall durch Mikroorganisem, dadurch gekennzeichnet, dass es neben üblichen Träger- und Hilfsstoffen als aktive Komponente mindestens eine Verbindung der Formel I

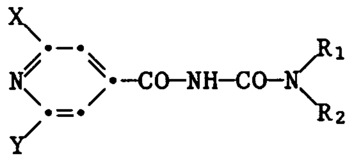

(I)

worin bedeuten:

X Wasserstoff oder Halogen;

Y Halogen;

$R_1$, $R_2$ unabhängig voneinander Wasserstoff, $C_1$-$C_6$-Alkyl, mit Halogen, Cyano, $C_1$-$C_4$-Alkoxy oder COO-$C_1$-$C_3$-Alkyl substituiertes $C_1$-$C_6$-Alkyl, $C_3$-$C_5$-Alkenyl, mit Halogen substituiertes $C_3$-$C_5$-Alkenyl, $C_3$-$C_5$-Alkinyl, mit Halogen substituiertes $C_3$-$C_5$-Alkinyl, $C_3$-$C_7$-Cycloalkyl, mit $C_1$-$C_2$-Alkyl, Halogen, Cyano oder COO-$C_1$-$C_3$-Alkyl substituiertes $C_3$-$C_7$-Cycloalkyl, Phenyl, Benzyl, mit $C_1$-$C_3$-Alkyl, $C_1$-$C_3$-Alkoxy oder Halogen substituiertes Phenyl oder Benzyl; und $R_1$ weiterhin auch den Rest Q oder mit $C_1$-$C_3$-Alkyl, $C_3$-$C_7$-Cycloalkyl, $C_1$-$C_3$-Alkoxy oder Halogen substituiertes Q darstellen kann, wobei Q über ein $C_1$-$C_2$-Alkyl an das N-Atom gebunden sein kann; oder worin $R_1$ und $R_2$ zusammen mit dem benachbarten N-Atom einen 3- bis 7-gliedrigen Heterocyclus bilden, der zusätzlich 1 oder 2 weitere Heteroatome, wie O, N oder S, und eine Carbonylgruppe enthalten und mit $C_1$-$C_3$-Alkyl, Halogen oder COO-$C_1$-$C_3$-Alkyl substituiert sein kann;

Q Furan-2-yl, Thiophen-2-yl, Isoxazol-3-yl, Isoxazol-5-yl, Thiazol-2-yl, 1,2,4-Thiadiazol-3-yl, 1,3,4-Thiadiazol-2-yl, 2-, 3- oder 4-Pyridylreste, mit $C_1$-$C_3$-Alkyl, $C_1$-$C_3$-Alkoxy, $C_1$-$C_3$-Alkylthio oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy, $C_1$-$C_3$-Alkylthio, Halogen oder Cyclopropyl substituierte 2-, 4- oder 5-Pyrimidylreste, entält.

**11.** Mittel gemäss Anspruch 10, dadurch gekennzeichnet, dass es als aktive Komponente mindestens eine Verbindung der Formel I

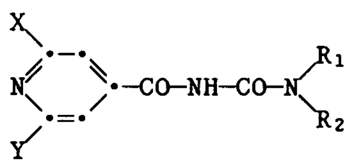

(I)

worin bedeuten:

X Wasserstoff oder Halogen;

Y Halogen;

$R_1$, $R_2$ unabhängig voneinander Wasserstoff, $C_1$-$C_6$-Alkyl, mit Halogen, Cyano oder COO-$C_1$-$C_3$-Alkyl substituiertes $C_1$-$C_6$-Alkyl, $C_3$-$C_5$-Alkenyl, mit Halogen substituiertes $C_3$-$C_5$-Alkenyl, $C_3$-$C_5$-Alkinyl, mit Halogen substituiertes $C_3$-$C_5$-Alkinyl, $C_3$-$C_7$-Cycloalkyl, mit $C_1$-$C_2$-Alkyl, Halogen, Cyano oder COO-$C_1$-$C_3$-Alkyl substituiertes $C_3$-$C_7$-Cycloalkyl, Benzyl, mit $C_1$-$C_3$-Alkyl, $C_1$-$C_3$-Alkoxy oder Halogen substituiertes Benzyl; und $R_1$ weiterhin auch den Rest Q oder mit $C_1$-$C_3$-Alkyl, $C_3$-$C_7$-Cycloalkyl, $C_1$-$C_3$-Alkoxy oder Halogen substituiertes Q darstellen kann, wobei Q über ein $C_1$-$C_2$-Alkyl an das N-Atom gebunden sein kann; oder worin $R_1$ und $R_2$ zusammen mit dem benachbarten N-Atom einen 3- bis 7-gliedrigen Heterocyclus bilden, der zusätzlich 1 oder 2 weitere Heteroatome, wie O, N oder S, und eine Carbonylgruppe enthalten und mit $C_1$-$C_3$-Alkyl, Halogen oder COO-$C_1$-$C_3$-Alkyl substituiert sein kann;

Q Furan-2-yl, Thiophen-2-yl, Isoxazol-3-yl, Isoxazol-5-yl, Thiazol-2-yl, 1,2,4-Thiadiazol-3-yl, 1,3,4-Thiadiazol-2-yl, 2-, 3- oder 4-Pyridylreste, mit $C_1$-$C_3$-Alkyl, $C_1$-$C_3$-Alkoxy, $C_1$-$C_3$-Alkylthio oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy, $C_1$-$C_3$-Alkylthio, Halogen oder Cyclopropyl substituierte 2-, 4- oder 5-Pyrimidylreste, enthält.

12. Mittel gemäss Anspruch 10, dadurch gekennzeichnet, dass es als aktive Komponente mindestens eine Verbindung der Formel I worin bedeuten:

| | |
|---|---|
| X | Wasserstoff, Fluor, Chlor oder Brom; |
| Y | Fluor, Chlor oder Brom; |
| $R_1$ | $C_1$-$C_6$-Alkyl, mit Fluor, Chlor, Cyano oder COO-$C_1$-$C_3$-Alkyl substituiertes $C_1$-$C_6$-Alkyl, $C_3$-$C_5$-Alkenyl, mit Chlor substituiertes Alkenyl, $C_3$-$C_5$-Alkinyl, mit Chlor substituiertes $C_3$-$C_5$-Alkinyl, $C_3$-$C_7$-Cycloalkyl, mit COO-$C_1$-$C_3$-Alkoxy substituiertes $C_3$-$C_7$-Cycloalkyl, Benzyl, mit $C_1$-$C_3$-Alkoxy oder Halogen substituiertes Benzyl oder Q; |
| $R_2$ | Wasserstoff, $C_1$-$C_6$-Alkyl oder mit Fluor, Chlor, Cyano oder COO-$C_1$-$C_3$-Alkyl substituiertes $C_1$-$C_6$-Alkyl; |
| $R_1$ und $R_2$ | bilden zusammen mit dem benachbarten N-Atom einen 4- bis 7-gliedrigen Heterocyclus, der zusätzlich ein weiteres N-Atom oder ein O-Atom enthalten und mit Methyl oder COO-$C_1$-$C_3$-Alkyl substituiert sein kann; |
| Q | Furan-2-yl, Thiophen-2-yl, Isoxazol-3-yl oder Isoxazol-5-yl, 2-, 3- oder 4-Pyridylreste, mit $C_1$-$C_3$-Alkyl, $C_1$-$C_3$-Alkoxy oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy oder Halogen substituierte 2-, 4- oder 5-Pyrimidylreste, enthält. |

13. Mittel gemäss Anspruch 10, dadurch gekennzeichnet, dass es als aktive Komponente mindestens eine Verbindung der Formel I worin bedeuten:

| | |
|---|---|
| X | Wasserstoff, Fluor, Chlor oder Brom; |
| Y | Fluor, Chlor oder Brom; |
| $R_1$ | $C_1$-$C_6$-Alkyl, mit Halogen substituiertes $C_1$-$C_6$-Alkyl oder Q; |
| $R_2$ | Wasserstoff, $C_1$-$C_6$-Alkyl oder mit Fluor, Chlor, Cyano oder COO-$C_1$-$C_3$-Alkyl substituiertes $C_1$-$C_6$-Alkyl; |
| $R_1$ und $R_2$ | bilden zusammen mit dem benachbarten N-Atom einen 4- bis 7-gliedrigen Heterocyclus, der zusätzlich ein N- oder ein O-Atom enthalten und mit Halogen substituiert sein kann; |
| Q | Thiazol-2-yl, 1,2,4-Thiazol-3-yl, 1,3,4-Thiadiazol-2-yl, 2-, 3- oder 4-Pyridylreste oder mit $C_1$-$C_3$-Alkyl, $C_1$-$C_3$-Alkoxy oder Halogen substituierte 2-, 3- oder 4-Pyridylreste, enthält. |

14. Mittel gemäss Anspruch 10, dadurch gekennzeichnet, dass es als aktive Komponente mindestens eine Verbindung der Formel I worin bedeuten:

| | |
|---|---|
| X | Wasserstoff, Chlor oder Brom; |
| Y | Chlor oder Brom; |
| $R_1$ | $C_1$-$C_4$-Alkyl, mit Chlor oder COO-$C_1$-$C_3$-Alkyl substituiertes $C_1$-$C_4$-Alkyl, $C_3$-$C_5$-Alkenyl, $C_3$-$C_5$-Alkinyl, Cyclopropyl, mit $COOCH_3$ substituiertes Cyclopropyl, Benzyl, mit Methoxy oder Chlor substituiertes Benzyl oder Q; |
| $R_2$ | Wasserstoff, $C_1$-$C_4$-Alkyl, mit Chlor oder COO-$C_1$-$C_3$-Alkyl substituiertes $C_1$-$C_4$-Alkyl; |
| $R_1$ und $R_2$ | bilden zusammen mit dem benachbarten N-Atom einen 5- oder 6-gliedrigen Heterocyclus, der zusätzlich ein weiteres N- oder ein O-Atom enthalten und mit Methyl oder COO-$C_1$-$C_3$-Alkyl substituiert sein kann; |
| Q | Furan-2-yl, Thiophen-2-yl, Isoxazol-3-yl, Isoxazol-5-yl, 2-, 3- oder 4-Pyridylreste, mit $C_1$-$C_3$-Alkyl, $C_1$-$C_3$-Alkoxy, Chlor oder Brom substituierte 2-, 3- oder 4-Pyridylreste; 2-, 4- oder 5-Pyrimidylreste oder mit $C_1$-$C_3$-Alkyl, $C_1$-$C_3$-Alkoxy, Chlor oder Brom substituierte 2-, 4- oder 5-Pyrimidylreste, enthält. |

15. Mittel gemäss Anspruch 10, dadurch gekennzeichnet, dass es als aktive Komponente mindestens eine Verbindung der Formel I worin bedeuten:

| | |
|---|---|
| X | Wasserstoff, Chlor oder Brom; |
| Y | Chlor oder Brom; |
| $R_1$ | $C_1$-$C_4$-Alkyl, mit Fluor oder Chlor substituiertes $C_1$-$C_4$-Alkyl oder Q; |
| $R_2$ | Wasserstoff, $C_1$-$C_4$-Alkyl, mit Chlor oder COO-$C_1$-$C_3$-Alkyl substituiertes $C_1$-$C_4$-Alkyl; |
| $R_1$ und $R_2$ | bilden zusammen mit dem benachbarten N-Atom einen 5- oder 6-gliedrigen Heterocyclus, der zusätzlich ein weiteres N-Atom oder ein O-Atom enthalten und mit Chlor oder Brom substituiert sein kann; |
| Q | Thiazol-2-yl, 1,2,4-Thiadiazol-3-yl, 1,3,4-Thiadiazol-2-yl, 2-, 3- oder 4-Pyridylreste oder |

mit Methyl, Methoxy, Fluor, Chlor oder Brom substituierte 2-, 3- oder 4-Pyridylreste, enthält.

**16.** Mittel gemäss Anspruch 10, dadurch gekennzeichnet, dass es als aktive Komponente die Verbindung N-(2,6-Dichloroisonicotinoyl)-carbamoyl-piperidin enthält.

**17.** Mittel gemäss Anspruch 10, dadurch gekennzeichnet, dass es als aktive Komponente die Verbindung N,N-Dimethyl-N'-(2,6-dichloroisonicotinoyl)-harnstoff enthält.

**18.** Verfahren zum Schutz von Pflanzen gegen den Befall durch phytopathogene Mikroorganismen, dadurch gekennzeichnet, dass man als Wirkstoff eine Verbindung der Formel I auf die Pflanze oder deren Standort appliziert,

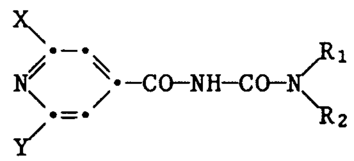

(I)

worin bedeuten:

X Wasserstoff oder Halogen;

Y Halogen;

$R_1$, $R_2$ unabhängig voneinander Wasserstoff, $C_1$-$C_6$-Alkyl, mit Halogen, Cyano, $C_1$-$C_4$-Alkoxy oder COO-$C_1$-$C_3$-Alkyl substituiertes $C_1$-$C_6$-Alkyl, $C_3$-$C_5$-Alkenyl, mit Halogen substituiertes $C_3$-$C_5$-Alkenyl, $C_3$-$C_5$-Alkinyl, mit Halogen substituiertes $C_3$-$C_5$-Alkinyl, $C_3$-$C_7$-Cycloalkyl, mit $C_1$-$C_2$-Alkyl, Halogen, Cyano oder COO-$C_1$-$C_3$-Alkyl substituiertes $C_3$-$C_7$-Cycloalkyl, Phenyl, Benzyl, mit $C_1$-$C_3$-Alkyl, $C_1$-$C_3$-Alkoxy oder Halogen substituiertes Phenyl oder Benzyl; und $R_1$ weiterhin auch den Rest Q oder mit $C_1$-$C_3$-Alkyl, $C_3$-$C_7$-Cycloalkyl, $C_1$-$C_3$-Alkoxy oder Halogen substituiertes Q darstellen kann, wobei Q über ein $C_1$-$C_2$-Alkyl an das N-Atom gebunden sein kann; oder worin $R_1$ und $R_2$ zusammen mit dem benachbarten N-Atom einen 3- bis 7-gliedrigen Heterocyclus bilden, der zusätzlich 1 oder 2 weitere Heteroatome, wie O, N oder S, und eine Carbonylgruppe enthalten und mit $C_1$-$C_3$-Alkyl, Halogen oder COO-$C_1$-$C_3$-Alkyl substituiert sein kann;

Q Furan-2-yl, Thiophen-2-yl, Isoxazol-3-yl, Isoxazol-5-yl, Thiazol-2-yl, 1,2,4-Thiadiazol-3-yl, 1,3,4-Thiadiazol-2-yl, 2-, 3- oder 4-Pyridylreste, mit $C_1$-$C_3$-Alkyl, $C_1$-$C_3$-Alkoxy, $C_1$-$C_3$-Alkylthio oder Halogen substituierte 2-, 3- oder 4-Pyridylreste; 2-, 4- oder 5-Pyrimidylreste oder mit $C_1$-$C_3$-Alkyl, $C_1$-$C_3$-Alkoxy, $C_1$-$C_3$-Alkylthio, Halogen oder Cyclopropyl substituierte 2-, 4- oder 5-Pyrimidylreste.

**19.** Verfahren zum Schutz von Pflanzen gegen den Befall durch phytopathogene Mikroorganismen, dadurch gekennzeichnet, dass als Wirkstoff eine Verbindung hergestellt wurde gemäss einem der Ansprüche 2 bis 9 auf die Pflanze oder deren Standort appliziert.

**Claims**

**Claims for the following Contracting States : AT, BE, CH, DE, FR, GB, GR, IT, LI, LU, NL, SE**

**1.** A compound of the formula I

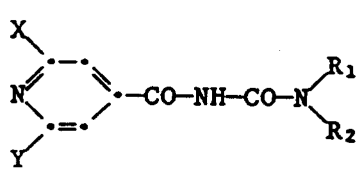

(I)

in which:

X is hydrogen or halogen; Y is halogen; $R_1$, $R_2$ independently of one another are hydrogen, $C_1$-$C_6$alkyl

or $C_1$-$C_6$alkyl which is substituted by halogen, cyano, $C_1$-$C_4$alkoxy or COO-$C_1$-$C_3$alkyl, or is $C_3$-$C_5$alkenyl or $C_3$-$C_5$alkenyl which is substituted by halogen, or is $C_3$-$C_5$alkynyl or $C_3$-$C_5$alkynyl which is substituted by halogen, or is $C_3$-$C_7$cycloalkyl or $C_3$-$C_7$cycloalkyl which is substituted by $C_1$-$C_2$alkyl, halogen, cyano or COO-$C_1$-$C_3$alkyl, or is phenyl, benzyl or phenyl or benzyl which are substituted by $C_1$-$C_3$alkyl, $C_1$-$C_3$alkoxy or halogen; and $R_1$ can furthermore also be the radical Q or Q which is substituted by $C_1$-$C_3$alkyl, $C_3$-$C_7$cycloalkyl, $C_1$-$C_3$alkoxy or halogen, where Q can be bonded to the N atom via a $C_1$-$C_2$alkyl; or where $R_1$ and $R_2$ together with the adjacent N atom form a 3- to 7-membered heterocycle which can additionally contain 1 or 2 further heteroatoms such as O, N or S, and a carbonyl group and can be substituted by $C_1$-$C_3$alkyl, halogen or COO-$C_1$-$C_3$alkyl; Q is furan-2-yl, thiophen-2-yl, isoxazol-3-yl, isoxazol-5-yl, thiazol-2-yl, 1,2,4-thiadiazol-3-yl, 1,3,4-thiadiazol-2-yl, 2-, 3- or 4-pyridyl radicals or 2-, 3- or 4-pyridyl radicals which are substituted by $C_1$-$C_3$alkyl, $C_1$-$C_3$alkoxy, $C_1$-$C_3$alkylthio or halogen; 2-, 4- or 5-pyrimidyl radicals or 2-, 4- or 5-pyrimidyl radicals which are substituted by $C_1$-$C_3$alkyl, $C_1$-$C_3$alkoxy, $C_1$-$C_3$alkylthio, halogen or cyclopropyl, with the exception of the compound 2,6-dichloro-N-(((4-chlorophenyl)amino)carbonyl)-4-pyridinecarboxamide.

**2.** A compound of the formula I

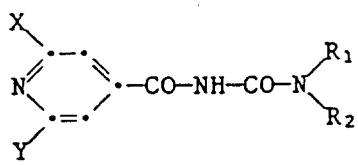

(I)

in which
X is hydrogen or halogen; Y is halogen; $R_1$, $R_2$ independently of one another are hydrogen, $C_1$-$C_6$alkyl or $C_1$-$C_6$alkyl which is substituted by halogen, cyano or COO-$C_1$-$C_3$alkyl, or is $C_3$-$C_5$alkenyl or $C_3$-$C_5$alkenyl which is substituted by halogen, or is $C_3$-$C_5$alkynyl or $C_3$-$C_5$alkynyl which is substituted by halogen, or is $C_3$-$C_7$cycloalkyl or $C_3$-$C_7$cycloalkyl which is substituted by $C_1$-$C_2$alkyl, halogen, cyano or COO-$C_1$-$C_3$alkyl, or is benzyl or benzyl which is substituted by $C_1$-$C_3$alkyl, $C_1$-$C_3$alkoxy or halogen; and $R_1$ can furthermore also be the radical Q or Q which is substituted by $C_1$-$C_3$alkyl, $C_3$-$C_7$cycloalkyl, $C_1$-$C_3$alkoxy or halogen, where Q can be bonded to the N atom via a $C_1$-$C_2$alkyl; or where $R_1$ and $R_2$ together with the adjacent N atom form a 3- to 7-membered heterocycle which can additionally contain 1 or 2 further heteroatoms such as O, N or S, and a carbonyl group and can be substituted by $C_1$-$C_3$alkyl, halogen or COO-$C_1$-$C_3$alkyl; Q is furan-2-yl, thiophen-2-yl, isoxazol-3-yl, isoxazol-5-yl, thiazol-2-yl, 1,2,4-thiadiazol-3-yl, 1,3,4-thiadiazol-2-yl, 2-, 3- or 4-pyridyl radicals, 2-, 3- or 4-pyridyl radicals which are substituted by $C_1$-$C_3$alkyl, $C_1$-$C_3$alkoxy, $C_1$-$C_3$alkylthio or halogen; 2-, 4- or 5-pyrimidyl radicals or 2-, 4- or 5-pyrimidyl radicals which are substituted by $C_1$-$C_3$alkyl, $C_1$-$C_3$alkoxy, $C_1$-$C_3$alkylthio, halogen or cyclopropyl.

**3.** A compound according to claim 2 where:
X is hydrogen, fluorine, chlorine or bromine; Y is fluorine, chlorine or bromine; $R_1$ is $C_1$-$C_6$alkyl or $C_1$-$C_6$alkyl which is substituted by fluorine, chlorine, cyano or COO-$C_1$-$C_3$alkyl, or is alkenyl or alkenyl which is substituted by chlorine, or is $C_3$-$C_5$alkynyl or $C_3$-$C_5$alkynyl which is substituted by chlorine, or is $C_3$-$C_7$cycloalkyl or $C_3$-$C_7$cycloalkyl which is substituted by COO-$C_1$-$C_3$alkoxy, or is benzyl or benzyl which is substituted by $C_1$-$C_3$alkoxy or halogen, or is Q; $R_2$ is hydrogen, $C_1$-$C_6$alkyl or $C_1$-$C_6$alkyl which is substituted by fluorine, chlorine, cyano or COO-$C_1$-$C_3$alkyl; $R_1$ and $R_2$ together with the adjacent N atom form a 4- to 7-membered heterocycle which can additionally contain a further N atom or an O atom and can be substituted by methyl or COO-$C_1$-$C_3$alkyl; Q is furan-2-yl, thiophen-2-yl, isoxazol-3-yl or isoxazol-5-yl, 2-, 3- or 4-pyridyl radicals or 2-, 3- or 4-pyridyl radicals which are substituted by $C_1$-$C_3$alkyl, $C_1$-$C_3$alkoxy or halogen; 2-, 4- or 5-pyrimidyl radicals or 2-, 4- or 5-pyrimidyl radicals which are substituted by $C_1$-$C_3$alkyl, $C_1$-$C_3$alkoxy or halogen.

**4.** A compound according to claim 2, where:
X is hydrogen, fluorine, chlorine or bromine; Y is fluorine, chlorine or bromine; $R_1$ is $C_1$-$C_6$alkyl or $C_1$-$C_6$alkyl which is substituted by halogen, or is Q; $R_2$ is hydrogen, $C_1$-$C_6$alkyl or $C_1$-$C_6$alkyl which is substituted by fluorine, chlorine, cyano or COO-$C_1$-$C_3$alkyl; $R_1$ and $R_2$ together with the adjacent N atom form a 4- to 7-membered heterocycle which can additionally contain an N- or an O-atom and can

be substituted by halogen; Q is thiazol-2-yl, 1,2,4-thiazol-3-yl, 1,3,4-thiadiazol-2-yl, 2-, 3- or 4-pyridyl radicals or 2-, 3- or 4-pyridyl radicals which are substituted by $C_1$-$C_3$alkyl, $C_1$-$C_3$alkoxy or halogen.

**5.** A compound according to claim 3, where:
X is hydrogen, chlorine or bromine; Y is chlorine or bromine; $R_1$ is $C_1$-$C_4$alkyl or $C_1$-$C_4$alkyl which is substituted by chlorine or COO-$C_1$-$C_3$alkyl, or is $C_3$-$C_5$alkenyl, $C_3$-$C_5$alkynyl, cyclopropyl or cyclopropyl which is substituted by $COOCH_3$, or is benzyl or benzyl which is substituted by methoxy or chlorine, or is Q; $R_2$ is hydrogen, $C_1$-$C_4$alkyl or $C_1$-$C_4$alkyl which is substituted by chlorine or COO-$C_1$-$C_3$alkyl; $R_1$ and $R_2$ together with the adjacent N atom form a 5- or 6-membered heterocycle which can additionally contain a further N or an O atom and can be substituted by methyl or COO-$C_1$-$C_3$alkyl; Q is furan-2-yl, thiophen-2-yl, isoxazol-3-yl, isoxazol-5-yl, 2-, 3- or 4-pyridyl radicals or 2-, 3- or 4-pyridyl radicals which are substituted by $C_1$-$C_3$alkyl, $C_1$-$C_3$alkoxy, chlorine or bromine; 2-, 4- or 5-pyrimidyl radicals or 2-, 4- or 5-pyrimidyl radicals which are substituted by $C_1$-$C_3$alkyl, $C_1$-$C_3$alkoxy, chlorine or bromine.

**6.** A compound according to claim 4, where:
X is hydrogen, chlorine or bromine; Y is chlorine or bromine; $R_1$ is $C_1$-$C_4$alkyl or $C_1$-$C_4$alkyl which is substituted by fluorine or chlorine, or is Q; $R_2$ is hydrogen, $C_1$-$C_4$alkyl or $C_1$-$C_4$alkyl which is substituted by chlorine or COO-$C_1$-$C_3$alkyl; $R_1$ and $R_2$ together with the adjacent N atom form a 5- or 6-membered heterocycle which can additionally contain a further N atom or an O atom and can be substituted by chlorine or bromine; Q is thiazol-2-yl, 1,2,4-thiadiazol-3-yl, 1,3,4-thiadiazol-2-yl, 2-, 3- or 4-pyridyl radicals or 2-, 3- or 4-pyridyl radicals which are substituted by methyl, methoxy, fluorine, chlorine or bromine.

**7.** N-(2,6-Dichloroisonicotinoyl)carbamoylpiperidine.

**8.** N,N-Dimethyl-N'-(2,6-dichloroisonicotinoyl)urea.

**9.** A process for the preparation of a compound of the formula I in claim 1, which comprises reacting:
an isonicotinamide of the formula II

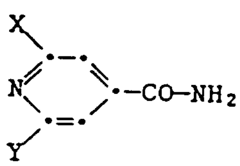

(II)

with

a) a carbamoyl halide of the formula IIIa

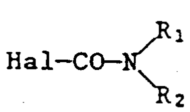

(IIIa)

or

b) a carbamoyl azolide of the formula IIIb

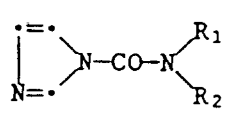

(IIIb)

or, for the preparation of those compounds of the formula I in which $R_1$ or $R_2$ is hydrogen,

c) with an isocyanate of the formula IIIc

$O=C=N\text{-}A$ (IIIc)

in the presence of a base in aprotic solvents, where Hal in the abovementioned compounds is halogen, preferably chlorine, A is as defined for $R_1$ and $R_2$, and this radical and X and Y are as defined under formula I.

**10.** A process for the preparation of a compound of the formula I in claim 1, which comprises allowing a compound of the formula II

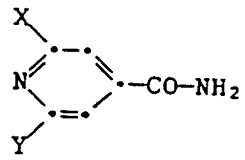

(II)

to react

a) with $(COCl)_2$ or $CO(Cl)_2$ to give an isocyanate of the formula IV

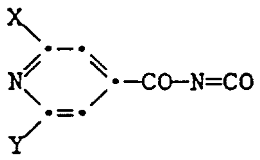

(IV)

which is then reacted

b) with an amine of the formula V

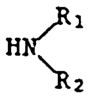

(V)

where the reaction steps take place in inert solvents.

**11.** An agent for protecting plants against infection with microorganisms, containing in addition to conventionally used carriers and auxiliaries at least one compound according to claim 1 as active component.

**12.** An agent according to claim 11, containing at least one compound according to any one of claims 2 to 6 as active component.

**13.** An agent according to claim 11, containing the compound of the formula I according to claim 7 as active component.

**14.** An agent according to claim 11, containing the compound of the formula I according to claim 8 as active component.

**15.** Use of compounds according to claim 1 for protecting plants against infection with phytopathogenic microorganisms.

**16.** Use of compounds according to claim 2 for protecting plants against infection with phytopathogenic microorganisms.

**Claims for the following Contracting State : ES**

**1.** A process for the preparation of a compound of the formula I

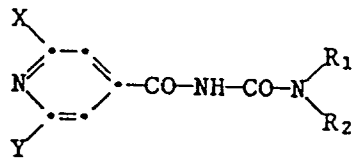

(I)

in which:
X is hydrogen or halogen; Y is halogen; $R_1$, $R_2$ independently of one another are hydrogen, $C_1$-$C_6$alkyl or $C_1$-$C_6$alkyl which is substituted by halogen, cyano, $C_1$-$C_4$alkoxy or COO-$C_1$-$C_3$alkyl, or is $C_3$-$C_5$alkenyl or $C_3$-$C_5$alkenyl which is substituted by halogen, or is $C_3$-$C_5$alkynyl or $C_3$-$C_5$alkynyl which is substituted by halogen, or is $C_3$-$C_7$cycloalkyl or $C_3$-$C_7$cycloalkyl which is substituted by $C_1$-$C_2$alkyl, halogen, cyano or COO-$C_1$-$C_3$alkyl, or is phenyl, benzyl or phenyl or benzyl which are substituted by $C_1$-$C_3$alkyl, $C_1$-$C_3$alkoxy or halogen; and $R_1$ can furthermore also be the radical Q or Q which is substituted by $C_1$-$C_3$alkyl, $C_3$-$C_7$cycloalkyl, $C_1$-$C_3$alkoxy or halogen, where Q can be bonded to the N atom via a $C_1$-$C_2$alkyl; or where $R_1$ and $R_2$ together with the adjacent N atom form a 3- to 7-membered heterocycle which can additionally contain 1 or 2 further heteroatoms such as O, N or S, and a carbonyl group and can be substituted by $C_1$-$C_3$alkyl, halogen or COO-$C_1$-$C_3$alkyl; Q is furan-2-yl, thiophen-2-yl, isoxazol-3-yl, isoxazol-5-yl, thiazol-2-yl, 1,2,4-thiadiazol-3-yl, 1,3,4-thiadiazol-2-yl, 2-, 3- or 4-pyridyl radicals or 2-, 3- or 4-pyridyl radicals which are substituted by $C_1$-$C_3$alkyl, $C_1$-$C_3$alkoxy, $C_1$-$C_3$alkylthio or halogen; 2-, 4- or 5-pyrimidyl radicals or 2-, 4- or 5-pyrimidyl radicals which are substituted by $C_1$-$C_3$alkyl, $C_1$-$C_3$alkoxy, $C_1$-$C_3$alkylthio, halogen or cyclopropyl, with the exception of the compound 2,6-dichloro-N-(((4-chlorophenyl)amino)carbonyl)-4-pyridinecarboxamide, which comprises reacting:
an isonicotinamide of the formula II with

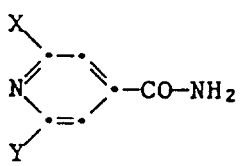

(II)

a) a carbamoyl halide of the formula IIIa

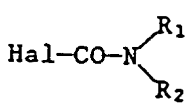

(IIIa)

or
b) a carbamoyl azolide of the formula IIIb

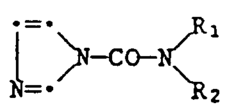

(IIIb)

or, for the preparation of those compounds of the formula I in which $R_1$ or $R_2$ is hydrogen,
c) with an isocyanate of the formula IIIc

O = C = N-A (IIIc)

in the presence of a base in aprotic solvents, where Hal in the abovementioned compounds is

halogen, preferably chlorine, A is as defined for $R_1$ and $R_2$, and this radical and X and Y are as defined under formula I.

**2.** A process for the preparation of a compound of the formula I

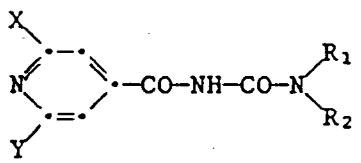

(I)

in which
X is hydrogen or halogen; Y is halogen; $R_1$, $R_2$ independently of one another are hydrogen, $C_1$-$C_6$alkyl or $C_1$-$C_6$alkyl which is substituted by halogen, cyano, $C_1$-$C_4$alkoxy or COO-$C_1$-$C_3$alkyl, or is $C_3$-$C_5$alkenyl or $C_3$-$C_5$alkenyl which is substituted by halogen, or is $C_3$-$C_5$alkynyl or $C_3$-$C_5$alkynyl which is substituted by halogen, or is $C_3$-$C_7$cycloalkyl or $C_3$-$C_7$cycloalkyl which is substituted by $C_1$-$C_2$alkyl, halogen, cyano or COO-$C_1$-$C_3$alkyl, or is phenyl, benzyl or phenyl or benzyl which is substituted by $C_1$-$C_3$alkyl, $C_1$-$C_3$alkoxy or halogen; and $R_1$ can furthermore also be the radical Q or Q which is substituted by $C_1$-$C_3$alkyl,
$C_3$-$C_7$cycloalkyl, $C_1$-$C_3$alkoxy or halogen, where Q can be bonded to the N atom via a $C_1$-$C_2$alkyl; or where $R_1$ and $R_2$ together with the adjacent N atom form a 3- to 7-membered heterocycle which can additionally contain 1 or 2 further heteroatoms such as O, N or S, and a carbonyl group and can be substituted by $C_1$-$C_3$alkyl, halogen or COO-$C_1$-$C_3$alkyl; Q is furan-2-yl, thiophen-2-yl, isoxazol-3-yl, isoxazol-5-yl, thiazol-2-yl, 1,2,4-thiadiazol-3-yl, 1,3,4-thiadiazol-2-yl, 2-, 3- or 4-pyridyl radicals, 2-, 3- or 4-pyridyl radicals which are substituted by $C_1$-$C_3$alkyl, $C_1$-$C_3$alkoxy, $C_1$-$C_3$alkylthio or halogen; 2-, 4- or 5-pyrimidyl radicals or 2-, 4- or 5-pyrimidyl radicals which are substituted by $C_1$-$C_3$alkyl, $C_1$-$C_3$alkoxy, $C_1$-$C_3$alkylthio, halogen or cyclopropyl, which comprises allowing a compound of the formula II

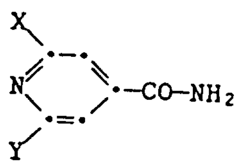

(II)

to react
a) with $(COCl)_2$ or $CO(Cl)_2$ to give an isocyanate of the formula IV

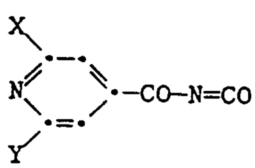

(IV)

which is then reacted
b) with an amine of the formula V

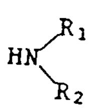

(V)

where the reaction steps take place in inert solvents and X, Y, $R_1$ and $R_2$ are as defined under formula I.

**3.** A process according to claim 1 or 2 which comprises preparing a compound of the formula I where: X is hydrogen or halogen; Y is halogen; $R_1$, $R_2$ independently of one another are hydrogen, $C_1$-$C_6$alkyl or $C_1$-$C_6$alkyl which is substituted by halogen, cyano or COO-$C_1$-$C_3$alkyl, or is $C_3$-$C_5$alkenyl or $C_3$-$C_5$alkenyl which is substituted by halogen, or is $C_3$-$C_5$alkynyl or $C_3$-$C_5$alkynyl which is substituted by halogen, or is $C_3$-$C_7$cycloalkyl or $C_3$-$C_7$cycloalkyl which is substituted by $C_1$-$C_2$alkyl, halogen, cyano or COO-$C_1$-$C_3$alkyl, or is benzyl or benzyl which is substituted by $C_1$-$C_3$alkyl, $C_1$-$C_3$alkoxy or halogen; and $R_1$ can furthermore also be the radical Q or Q which is substituted by $C_1$-$C_3$alkyl, $C_3$-$C_7$cycloalkyl, $C_1$-$C_3$alkoxy or halogen, where Q can be bonded to the N atom via a $C_1$-$C_2$alkyl; or where $R_1$ and $R_2$ together with the adjacent N atom form a 3- to 7-membered heterocycle which can additionally contain 1 or 2 further heteroatoms such as O, N or S, and a carbonyl group and can be substituted by $C_1$-$C_3$alkyl, halogen or COO-$C_1$-$C_3$alkyl; Q is furan-2-yl, thiophen-2-yl, isoxazol-3-yl, isoxazol-5-yl, thiazol-2-yl, 1,2,4-thiadiazol-3-yl, 1,3,4-thiadiazol-2-yl, 2-, 3- or 4-pyridyl radicals, 2-, 3- or 4-pyridyl radicals which are substituted by $C_1$-$C_3$alkyl, $C_1$-$C_3$alkoxy, $C_1$-$C_3$alkylthio or halogen; 2-, 4- or 5-pyrimidyl radicals or 2-, 4- or 5-pyrimidyl radicals which are substituted by $C_1$-$C_3$alkyl, $C_1$-$C_3$alkoxy, $C_1$-$C_3$alkylthio, halogen or cyclopropyl.

**4.** A process according to claim 1 or 2 which comprises preparing a compound of the formula I where: X is hydrogen, fluorine, chlorine or bromine; Y is fluorine, chlorine or bromine; $R_1$ is $C_1$-$C_6$alkyl or $C_1$-$C_6$alkyl which is substituted by fluorine, chlorine, cyano or COO-$C_1$-$C_3$alkyl, or is $C_3$-$C_5$alkenyl or alkenyl which is substituted by chlorine, or is $C_3$-$C_5$alkynyl or $C_3$-$C_5$alkynyl which is substituted by chlorine, or is $C_3$-$C_7$cycloalkyl or $C_3$-$C_7$cycloalkyl which is substituted by COO-$C_1$-$C_3$alkoxy, or is benzyl or benzyl which is substituted by $C_1$-$C_3$alkoxy or halogen, or is Q; $R_2$ is hydrogen, $C_1$-$C_6$alkyl or $C_1$-$C_6$alkyl which is substituted by fluorine, chlorine, cyano or COO-$C_1$-$C_3$alkyl; $R_1$ and $R_2$ together with the adjacent N atom form a 4- to 7-membered heterocycle which can additionally contain a further N atom or an O atom and can be substituted by methyl or COO-$C_1$-$C_3$alkyl; Q is furan-2-yl, thiophen-2-yl, isoxazol-3-yl or isoxazol-5-yl, 2-, 3- or 4-pyridyl radicals or 2-, 3- or 4-pyridyl radicals which are substituted by $C_1$-$C_3$alkyl, $C_1$-$C_3$alkoxy or halogen; 2-, 4- or 5-pyrimidyl radicals or 2-, 4- or 5-pyrimidyl radicals which are substituted by $C_1$-$C_3$alkyl, $C_1$-$C_3$alkoxy or halogen.

**5.** A process according to claim 1 or 2, which comprises preparing a compound of the formula I where: X is hydrogen, fluorine, chlorine or bromine; Y is fluorine, chlorine or bromine; $R_1$ is $C_1$-$C_6$alkyl or $C_1$-$C_6$alkyl which is substituted by halogen, or is Q; $R_2$ is hydrogen, $C_1$-$C_6$alkyl or $C_1$-$C_6$alkyl which is substituted by fluorine, chlorine, cyano or COO-$C_1$-$C_3$alkyl; $R_1$ and $R_2$ together with the adjacent N atom form a 4- to 7-membered heterocycle which can additionally contain an N- or an O-atom and can be substituted by halogen; Q is thiazol-2-yl, 1,2,4-thiazol-3-yl, 1,3,4-thiadiazol-2-yl, 2-, 3- or 4-pyridyl radicals or 2-, 3- or 4-pyridyl radicals which are substituted by $C_1$-$C_3$alkyl, $C_1$-$C_3$alkoxy or halogen.

**6.** A process according to claim 1 or 2, which comprises preparing a compound of the formula I where: X is hydrogen, chlorine or bromine; Y is chlorine or bromine; $R_1$ is $C_1$-$C_4$alkyl or $C_1$-$C_4$alkyl which is substituted by chlorine or COO-$C_1$-$C_3$alkyl, or is $C_3$-$C_5$alkenyl, $C_3$-$C_5$alkynyl, cyclopropyl or cyclopropyl which is substituted by $COOCH_3$, or is benzyl or benzyl which is substituted by methoxy or chlorine, or is Q; $R_2$ is hydrogen, $C_1$-$C_4$alkyl or $C_1$-$C_4$alkyl which is substituted by chlorine or COO-$C_1$-$C_3$alkyl; $R_1$ and $R_2$ together with the adjacent N atom form a 5- or 6-membered heterocycle which can additionally contain a further N or an O atom and can be substituted by methyl or COO-$C_1$-$C_3$alkyl; Q is furan-2-yl, thiophen-2-yl, isoxazol-3-yl, isoxazol-5-yl, 2-, 3- or 4-pyridyl radicals or 2-, 3- or 4-pyridyl radicals which are substituted by $C_1$-$C_3$alkyl, $C_1$-$C_3$alkoxy, chlorine or bromine; 2-, 4- or 5-pyrimidyl radicals or 2-, 4- or 5-pyrimidyl radicals which are substituted by $C_1$-$C_3$alkyl, $C_1$-$C_3$alkoxy, chlorine or bromine.

**7.** A process according to claim 1 or 2, which comprises preparing a compound of the formula I where: X is hydrogen, chlorine or bromine; Y is chlorine or bromine; $R_1$ is $C_1$-$C_4$alkyl or $C_1$-$C_4$alkyl which is substituted by fluorine or chlorine, or is Q; $R_2$ is hydrogen, $C_1$-$C_4$alkyl or $C_1$-$C_4$alkyl which is substituted by chlorine or COO-$C_1$-$C_3$alkyl; $R_1$ and $R_2$ together with the adjacent N atom form a 5- or 6-membered heterocycle which can additionally contain a further N atom or an O atom and can be substituted by chlorine or bromine; Q is thiazol-2-yl, 1,2,4-thiadiazol-3-yl, 1,3,4-thiadiazol-2-yl, 2-, 3- or 4-pyridyl radicals or 2-, 3- or 4-pyridyl radicals which are substituted by methyl, methoxy, fluorine, chlorine or bromine.

8. A process according to claim 1 or 2, which comprises preparing N-(2,6-dichloroisonicotinoyl)-carbamoylpiperidine.

9. A process according to claim 1 or 2, which comprises preparing N,N-dimethyl-N'-(2,6-dichloroisonicotinoyl)urea.

10. An agent for protecting plants against infection with microorganisms, containing as active component in addition to conventionally used carriers and auxiliaries at least one compound of the formula I

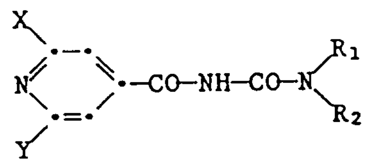

(I)

in which:
X is hydrogen or halogen; Y is halogen; $R_1$, $R_2$ independently of one another are hydrogen, $C_1$-$C_6$alkyl or $C_1$-$C_6$alkyl which is substituted by halogen, cyano, $C_1$-$C_4$alkoxy or COO-$C_1$-$C_3$alkyl, or is $C_3$-$C_5$alkenyl or $C_3$-$C_5$alkenyl which is substituted by halogen, or is $C_3$-$C_5$alkynyl or $C_3$-$C_5$alkynyl which is substituted by halogen, or is $C_3$-$C_7$cycloalkyl or $C_3$-$C_7$cycloalkyl which is substituted by $C_1$-$C_2$alkyl, halogen, cyano or COO-$C_1$-$C_3$alkyl, or is phenyl, benzyl or phenyl or benzyl which are substituted by $C_1$-$C_3$alkyl, $C_1$-$C_3$alkoxy or halogen; and $R_1$ can furthermore also be the radical Q or Q which is substituted by $C_1$-$C_3$alkyl, $C_3$-$C_7$cycloalkyl, $C_1$-$C_3$alkoxy or halogen, where Q can be bonded to the N atom via a $C_1$-$C_2$alkyl; or where $R_1$ and $R_2$ together with the adjacent N atom form a 3- to 7-membered heterocycle which can additionally contain 1 or 2 further heteroatoms such as O, N or S, and a carbonyl group and can be substituted by $C_1$-$C_3$alkyl, halogen or COO-$C_1$-$C_3$alkyl; Q is furan-2-yl, thiophen-2-yl, isoxazol-3-yl, isoxazol-5-yl, thiazol-2-yl, 1,2,4-thiadiazol-3-yl, 1,3,4-thiadiazol-2-yl, 2-, 3- or 4-pyridyl radicals or 2-, 3- or 4-pyridyl radicals which are substituted by $C_1$-$C_3$alkyl, $C_1$-$C_3$alkoxy, $C_1$-$C_3$alkylthio or halogen; 2-, 4- or 5-pyrimidyl radicals or 2-, 4- or 5-pyrimidyl radicals which are substituted by $C_1$-$C_3$alkyl, $C_1$-$C_3$alkoxy, $C_1$-$C_3$alkylthio, halogen or cyclopropyl.

11. An agent according to claim 10, containing as active component at least one compound of the formula I

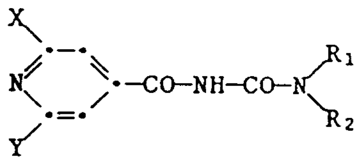

(I)

in which
X is hydrogen or halogen; Y is halogen; $R_1$, $R_2$ independently of one another are hydrogen, $C_1$-$C_6$alkyl or $C_1$-$C_6$alkyl which is substituted by halogen, cyano or COO-$C_1$-$C_3$alkyl, or is $C_3$-$C_5$alkenyl or $C_3$-$C_5$alkenyl which is substituted by halogen, or is $C_3$-$C_5$alkynyl or $C_3$-$C_5$alkynyl which is substituted by halogen, or is $C_3$-$C_7$cycloalkyl or $C_3$-$C_7$cycloalkyl which is substituted by $C_1$-$C_2$alkyl, halogen, cyano or COO-$C_1$-$C_3$alkyl, or is benzyl or benzyl which is substituted by $C_1$-$C_3$alkyl, $C_1$-$C_3$alkoxy or halogen; and $R_1$ can furthermore also be the radical Q or Q which is substituted by $C_1$-$C_3$alkyl, $C_3$-$C_7$cycloalkyl, $C_1$-$C_3$alkoxy or halogen, where Q can be bonded to the N atom via a $C_1$-$C_2$alkyl; or where $R_1$ and $R_2$ together with the adjacent N atom form a 3- to 7-membered heterocycle which can additionally contain 1 or 2 further heteroatoms such as O, N or S, and a carbonyl group and can be substituted by $C_1$-$C_3$alkyl, halogen or COO-$C_1$-$C_3$alkyl; Q is furan-2-yl, thiophen-2-yl, isoxazol-3-yl, isoxazol-5-yl, thiazol-2-yl, 1,2,4-thiadiazol-3-yl, 1,3,4-thiadiazol-2-yl, 2-, 3- or 4-pyridyl radicals, 2-, 3- or 4-pyridyl radicals which are substituted by $C_1$-$C_3$alkyl, $C_1$-$C_3$alkoxy, $C_1$-$C_3$alkylthio or halogen; 2-, 4- or 5-pyrimidyl radicals or 2-, 4- or 5-pyrimidyl radicals which are substituted by $C_1$-$C_3$alkyl, $C_1$-$C_3$alkoxy, $C_1$-$C_3$alkylthio, halogen or cyclopropyl.

**12.** An agent according to claim 10, containing as active component at least one compound of the formula I where:
X is hydrogen, fluorine, chlorine or bromine; Y is fluorine, chlorine or bromine; $R_1$ is $C_1$-$C_6$alkyl or $C_1$-$C_6$alkyl which is substituted by fluorine, chlorine, cyano or COO-$C_1$-$C_3$alkyl, or is $C_3$-$C_5$alkenyl or alkenyl which is substituted by chlorine, or is $C_3$-$C_5$alkynyl or $C_3$-$C_5$alkynyl which is substituted by chlorine, or is $C_3$-$C_7$cycloalkyl or $C_3$-$C_7$cycloalkyl which is substituted by COO-$C_1$-$C_3$alkoxy, or is benzyl or benzyl which is substituted by $C_1$-$C_3$alkoxy or halogen, or is Q; $R_2$ is hydrogen, $C_1$-$C_6$alkyl or $C_1$-$C_6$alkyl which is substituted by fluorine, chlorine, cyano or COO-$C_1$-$C_3$alkyl; $R_1$ and $R_2$ together with the adjacent N atom form a 4- to 7-membered heterocycle which can additionally contain a further N atom or an O atom and can be substituted by methyl or COO-$C_1$-$C_3$alkyl; Q is furan-2-yl, thiophen-2-yl, isoxazol-3-yl or isoxazol-5-yl, 2-, 3- or 4-pyridyl radicals or 2-, 3- or 4-pyridyl radicals which are substituted by $C_1$-$C_3$alkyl, $C_1$-$C_3$alkoxy or halogen; 2-, 4- or 5-pyrimidyl radicals or 2-, 4- or 5-pyrimidyl radicals which are substituted by $C_1$-$C_3$alkyl, $C_1$-$C_3$alkoxy or halogen.

**13.** An agent according to claim 10, containing as active component at least one compound of the formula I where:
X is hydrogen, fluorine, chlorine or bromine; Y is fluorine, chlorine or bromine; $R_1$ is $C_1$-$C_6$alkyl or $C_1$-$C_6$alkyl which is substituted by halogen, or is Q; $R_2$ is hydrogen, $C_1$-$C_6$alkyl or $C_1$-$C_6$alkyl which is substituted by fluorine, chlorine, cyano or COO-$C_1$-$C_3$alkyl; $R_1$ and $R_2$ together with the adjacent N atom form a 4- to 7-membered heterocycle which can additionally contain an N- or an O-atom and can be substituted by halogen; Q is thiazol-2-yl, 1,2,4-thiazol-3-yl, 1,3,4-thiadiazol-2-yl, 2-, 3- or 4-pyridyl radicals or 2-, 3- or 4-pyridyl radicals which are substituted by $C_1$-$C_3$alkyl, $C_1$-$C_3$alkoxy or halogen.

**14.** An agent according to claim 10, containing as active component at least one compound of the formula I where:
X is hydrogen, chlorine or bromine; Y is chlorine or bromine; $R_1$ is $C_1$-$C_4$alkyl or $C_1$-$C_4$alkyl which is substituted by chlorine or COO-$C_1$-$C_3$alkyl, or is $C_3$-$C_5$alkenyl, $C_3$-$C_5$alkynyl, cyclopropyl or cyclopropyl which is substituted by $COOCH_3$, or is benzyl or benzyl which is substituted by methoxy or chlorine, or is Q; $R_2$ is hydrogen, $C_1$-$C_4$alkyl or $C_1$-$C_4$alkyl which is substituted by chlorine or COO-$C_1$-$C_3$alkyl; $R_1$ and $R_2$ together with the adjacent N atom form a 5- or 6-membered heterocycle which can additionally contain a further N or an O atom and can be substituted by methyl or COO-$C_1$-$C_3$alkyl; Q is furan-2-yl, thiophen-2-yl, isoxazol-3-yl, isoxazol-5-yl, 2-, 3- or 4-pyridyl radicals or 2-, 3- or 4-pyridyl radicals which are substituted by $C_1$-$C_3$alkyl, $C_1$-$C_3$alkoxy, chlorine or bromine; 2-, 4- or 5-pyrimidyl radicals or 2-, 4- or 5-pyrimidyl radicals which are substituted by $C_1$-$C_3$alkyl, $C_1$-$C_3$alkoxy, chlorine or bromine.

**15.** An agent according to claim 10, containing as active component at least one compound of the formula I where:
X is hydrogen, chlorine or bromine; Y is chlorine or bromine; $R_1$ is $C_1$-$C_4$alkyl or $C_1$-$C_4$alkyl which is substituted by fluorine or chlorine, or is Q; $R_2$ is hydrogen, $C_1$-$C_4$alkyl or $C_1$-$C_4$alkyl which is substituted by chlorine or COO-$C_1$-$C_3$alkyl; $R_1$ and $R_2$ together with the adjacent N atom form a 5- or 6-membered heterocycle which can additionally contain a further N atom or an O atom and can be substituted by chlorine or bromine; Q is thiazol-2-yl, 1,2,4-thiadiazol-3-yl, 1,3,4-thiadiazol-2-yl, 2-, 3- or 4-pyridyl radicals or 2-, 3- or 4-pyridyl radicals which are substituted by methyl, methoxy, fluorine, chlorine or bromine.

**16.** An agent according to claim 10, containing as active component the compound N-(2,6-dichloroisonicotinoyl)carbamoylpiperidine.

**17.** An agent according to claim 10, containing as active component the compound N,N-dimethyl-N'-(2,6-dichloroisonicotinoyl)urea.

**18.** A method of protecting plants against infection with phytopathogenic microorganisms which comprises applying, as active substance, a compound of the formula I to the plant or its location

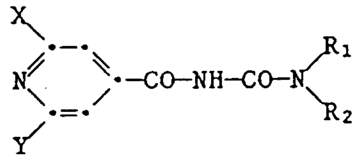

(I)

in which:
X is hydrogen or halogen; Y is halogen; $R_1$, $R_2$ independently of one another are hydrogen, $C_1$-$C_6$alkyl or $C_1$-$C_6$alkyl which is substituted by halogen, cyano, $C_1$-$C_4$alkoxy or COO-$C_1$-$C_3$alkyl, or is $C_3$-$C_5$alkenyl or $C_3$-$C_5$alkenyl which is substituted by halogen, or is $C_3$-$C_5$alkynyl or $C_3$-$C_5$alkynyl which is substituted by halogen, or is $C_3$-$C_7$cycloalkyl or $C_3$-$C_7$cycloalkyl which is substituted by $C_1$-$C_2$alkyl, halogen, cyano or COO-$C_1$-$C_3$alkyl, or is phenyl, benzyl or phenyl or benzyl which are substituted by $C_1$-$C_3$alkyl, $C_1$-$C_3$alkoxy or halogen; and $R_1$ can furthermore also be the radical Q or Q which is substituted by $C_1$-$C_3$alkyl, $C_3$-$C_7$cycloalkyl, $C_1$-$C_3$alkoxy or halogen, where Q can be bonded to the N atom via a $C_1$-$C_2$alkyl; or where $R_1$ and $R_2$ together with the adjacent N atom form a 3- to 7-membered heterocycle which can additionally contain 1 or 2 further heteroatoms such as O, N or S, and a carbonyl group and can be substituted by $C_1$-$C_3$alkyl, halogen or COO-$C_1$-$C_3$alkyl; Q is furan-2-yl, thiophen-2-yl, isoxazol-3-yl, isoxazol-5-yl, thiazol-2-yl, 1,2,4-thiadiazol-3-yl, 1,3,4-thiadiazol-2-yl, 2-, 3- or 4-pyridyl radicals or 2-, 3- or 4-pyridyl radicals which are substituted by $C_1$-$C_3$alkyl, $C_1$-$C_3$alkoxy, $C_1$-$C_3$alkylthio or halogen; 2-, 4- or 5-pyrimidyl radicals or 2-, 4- or 5-pyrimidyl radicals which are substituted by $C_1$-$C_3$alkyl, $C_1$-$C_3$alkoxy, $C_1$-$C_3$alkylthio, halogen or cyclopropyl.

**19.** A method of protecting plants against infection with phytopathogenic microorganisms, which comprises applying, as active substance, a compound prepared according to any one of claims 2 to 9 to the plant or its location.

**Revendications**
**Revendications pour les Etats contractants suivants : AT, BE, CH, DE, FR, GB, GR, IT, LI, LU, NL, SE**

**1.** Composés de formule I

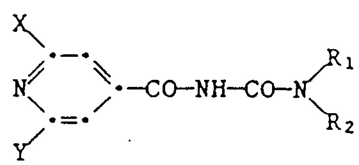

(I)

dans laquelle

X représente l'hydrogène ou un halogène;

Y représente un halogène;

$R_1$, $R_2$ représentent chacun, indépendamment l'un de l'autre, l'hydrogène, un groupe alkyle en C1-C6, un groupe alkyle en C1-C6 substitué par des halogènes, des groupes cyano, alcoxy en C1-C4 ou COO-alkyle en C1-C3, un groupe alcényle en C3-C5, un groupe alcényle en C3-C5 substitué par des halogènes, un groupe alcynyle en C3-C5, un groupe alcynyle en C3-C5 substitué par des halogènes, un groupe cycloalkyle en C3-C7, un groupe cycloalkyle en C3-C7 substitué par des groupes alkyle en C1-C2, des halogènes, des groupes cyano ou COO-alkyle en C1-C3, un groupe phényle, benzyle, un groupe phényle ou benzyle substitué par des groupes alkyle en C1-C3, alcoxy en C1-C3 ou des halogènes; $R_1$ pouvant en outre représenter le groupe Q ou le groupe Q substitué par des groupes alkyle en C1-C3, cycloalkyle en C3-C7, alcoxy en C1-C3 ou des halogènes, Q pouvant être relié à l'atome d'azote par l'intermédiaire d'un groupe alkyle en C1-C2; ou bien $R_1$ et $R_2$ forment ensemble et avec l'atome d'azote voisin un hétérocycle de 3 à 7 chaînons qui peut contenir 1 ou 2 autres hétéroatomes tels que O, N ou S, et un groupe carbonyle, et qui peut être substitué par des groupes alkyle en C1-C3, des halogènes ou des groupes COO-alkyle en C1-C3;

Q représente un groupe furanne-2-yle, thiophène-2-yle, isoxazole-3-yle, isoxazole-5-yle,

thiazole-2-yle, 1,2,4-thiadiazole-3-yle, 1,3,4-thiadiazole-2-yle, 2-, 3- ou 4-pyridyle, 2-, 3- ou 4-pyridyle substitué par des groupes alkyle en C1-C3, alcoxy en C1-C3, alkylthio en C1-C3 ou des halogènes; un groupe 2-, 4- ou 5-pyrimidyle ou un groupe 2-, 4- ou 5-pyrimidyle substitué par des groupes alkyle en C1-C3, alcoxy en C1-C3, alkylthio en C1-C3, des halogènes ou des groupes cyclopropyle, à l'exception du composé 2,6-dichloro-N-(((4-chlorophényl)-amino)-carbonyl)-4-pyridine-carboxamide.

**2.** Composés de formule I

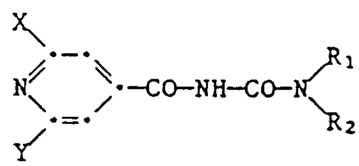

(I)

dans laquelle

X représente l'hydrogène ou un halogène;

Y représente un halogène;

$R_1$, $R_2$ représentent chacun, indépendamment l'un de l'autre, l'hydrogène, un groupe alkyle en C1-C6, un groupe alkyle en C1-C6 substitué par des halogènes, des groupes cyano ou COO-alkyle en C1-C3, un groupe alcényle en C3-C5, un groupe alcényle en C3-C5 substitué par des halogènes, un groupe alcynyle en C3-C5, un groupe alcynyle en C3-C5 substitué par des halogènes, un groupe cycloalkyle en C3-C7, un groupe cycloalkyle en C3-C7 substitué par des groupes alkyle en C1-C2, des halogènes, des groupes cyano ou COO-alkyle en C1-C3, un groupe benzyle, un groupe benzyle substitué par des groupes alkyle en C1-C3, alcoxy en C1-C3 ou des halogènes; et $R_1$ peut en outre représenter le groupe Q ou le groupe Q substitué par des groupes alkyle en C1-C3, cycloalkyle en C3-C7, alcoxy en C1-C3 ou des halogènes, Q pouvant être relié a l'atome d'azote par l'intermédiaire d'un groupe alkyle en C1-C2; ou bien $R_1$ et $R_2$ forment ensemble et avec l'atome d'azote voisin un hétérocycle de 3 à 7 chaînons qui peut en outre contenir 1 ou 2 autres hétéroatomes tels que O, N ou S et un groupe carbonyle et qui peut être substitué par des groupes alkyle en C1-C3, des halogènes ou des groupes COO-alkyle en C1-C3;

Q représente un groupe furanne-2-yle, thiophène-2-yle, isoxazole-3-yle, isoxazole-5-yle, thiazole-2-yle, 1,2,4-thiadiazole-3-yle, 1,3,4-thiadiazole-2-yle, 2-, 3- ou 4-pyridyle, 2-, 3- ou 4-pyridyle substitué par des groupes alkyle en C1-C3, alcoxy en C1-C3, alkylthio en C1-C3 ou des halogènes; un groupe 2-, 4- ou 5-pyrimidyle ou un groupe 2-, 4- ou 5-pyrimidyle substitué par des groupes alkyle en C1-C3, alcoxy en C1-C3, alkylthio en C1-C3, des halogènes ou des groupes cyclopropyle.

**3.** Composés selon revendication 2, pour lesquels :

X représente l'hydrogene, le fluor, le chlore ou le brome;

Y représente le fluor, le chlore ou le brome;

$R_1$ représente un groupe alkyle en C1-C6, un groupe alkyle en C1-C6 substitué par le fluor, le chlore, des groupes cyano ou COO-alkyle en C1-C3, un groupe alcényle en C3-C5, un groupe alcényle substitué par le chlore, un groupe alcynyle en C3-C5, un groupe alcynyle en C3-C5 substitué par le chlore, un groupe cycloalkyle en C3-C7, un groupe cycloalkyle en C3-C7 substitué par un groupe COO-alcoxy en C1-C3, un groupe benzyle, un groupe benzyle substitué par des groupes alcoxy en C1-C3 ou des halogènes, ou un groupe Q;

$R_2$ représente l'hydrogène, un groupe alkyle en C1-C6 ou un groupe alkyle en C1-C6 substitué par le fluor, le chlore, des groupes cyano ou COO-alkyle en C1-C3;

$R_1$ et $R_2$ forment ensemble et avec l'atome d'azote voisin un hétérocycle de 4 à 7 chaînons qui peut contenir encore un autre atome d'azote ou un atome d'oxygène et qui peint être substitué par des groupes méthyle ou COO-alkyle en C1-C3;

Q représente un groupe furanne-2-yle, thiophène-2-yle, isoxazole-3-yle ou isoxazole-5-yle, 2-, 3- ou 4-pyridyle, 2-, 3- ou 4-pyridyle substitué par des groupes alkyle en C1-C3, alcoxy en C1-C3 ou des halogènes; 2-, 4- ou 5-pyrimidyle ou 2-, 4- ou 5-pyrimidyle

substitué par des groupes alkyle en C1-C3, alcoxy en C1-C3 ou des halogènes.

**4.** Composés selon revendication 2, pour lesquels :

| | |
|---|---|
| X | représente l'hydrogène, le fluor, le chlore ou le brome; |
| Y | représente le fluor, le chlore ou le brome; |
| $R_1$ | représente un groupe alkyle en C1-C6, un groupe alkyle en C1-C6 substitué par des halogènes ou un groupe Q; |
| $R_2$ | représente l'hydrogène, un groupe alkyle en C1-C6, ou un groupe alkyle en C1-C6 substitué par le fluor, le chlore, des groupes cyano ou COO-alkyle en C1-C3; |
| $R_1$ et $R_2$ | forment ensemble et avec l'atome d'azote voisin un hétérocycle de 4 à 7 chaînons qui peut contenir un autre atome d'azote ou un atome d'oxygène et qui peut être substitué par des halogènes; |
| Q | représente un groupe thiazole-2-yle, 1,2,4-thiazole-3-yle, 1,3,4-thiazole-2-yle, 2-, 3- ou 4-pyridyle, 2-, 3- ou 4-pyridyle substitué par des groupes alkyle en C1-C3, alcoxy en C1-C3 ou des halogènes. |

**5.** Composés selon revendication 3, pour lesquels :

| | |
|---|---|
| X | représente l'hydrogène, le chlore ou le brome; |
| Y | représente le chlore ou le brome; |
| $R_1$ | représente un groupe alkyle en C1-C4, un groupe alkyle en C1-C4 substitué par le chlore ou des groupes COO-alkyle en C1-C3, un groupe alcényle en C3-C5, alcynyle en C3-C5, cyclopropyle, cyclopropyle substitué par $COOCH_3$, benzyle, benzyle substitué par des groupes méthoxy ou le chlore; ou Q; |
| $R_2$ | représente l'hydrogène, un groupe alkyle en C1-C4, un groupe alkyle en C1-C4 substitué par le chlore ou des groupes COO-alkyle en C1-C3; |
| $R_1$ et $R_2$ | forment ensemble et avec l'atome d'azote voisin un hétérocycle à 5 ou 6 chaînons qui peut contenir un autre atome d'azote et un atome d'oxygène et qui peut être substitué par des groupes méthyle ou COO-alkyle en C1-C3; |
| Q | représente un groupe furanne-2-yle, thiophène-2-yle, isoxazole-3-yle, isoxazole-5-yle, 2-, 3- ou 4-pyridyle, 2-, 3- ou 4-pyridyle substitué par des groupes alkyle en C1-C3, alcoxy en C1-C3, le chlore ou le brome; un groupe 2-, 4- ou 5-pyrimidyle ou un groupe 2-, 4- ou 5-pyrimidyle substitué par des groupes alkyle en C1-C3, alcoxy en C1-C3, le chlore ou le brome. |

**6.** Composés selon revendication 4, pour lesquels :

| | |
|---|---|
| X | représente l'hydrogène, le chlore ou le brome; |
| Y | représente le chlore ou le brome; |
| $R_1$ | représente un groupe alkyle en C1-C4, un groupe alkyle en C1-C4 substitué par le fluor ou le chlore ou un groupe Q; |
| $R_2$ | représente l'hydrogène, un groupe alkyle en C1-C4, un groupe alkyle en C1-C4 substitué par le chlore ou des groupes COO-alkyle en C1-C3; |
| $R_1$ et $R_2$ | forment ensemble et avec l'atome d'azote voisin un hétérocycle à 5 ou 6 chaînons qui peut encore contenir un autre atome d'azote ou un atome d'oxygène et qui peut être substitué par le chlore ou le brome; |
| Q | représente un groupe thiazole-2-yle, 1,2,4-thiadiazole-3-yle, 1,3,4-thiadiazole-2-yle, 2-, 3- ou 4-pyridyle, ou 2-, 3- ou 4-pyridyle substitué par des groupes méthyle, méthoxy, le fluor, le chlore ou le brome. |

**7.** La N-(2,6-dichlorisonicotinoyl)-carbamoyl-pipéridine.

**8.** La N,N-diméthyl-N'-(2,6-dichlorisonicotinoyl)-urée.

**9.** Procédé de préparation des composés de formule I de la revendication 1, caractérisé en ce que l'on fait réagir :
un isonicotinamide de formule II

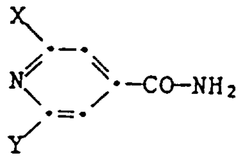

(II)

avec

a) un halogénure de carbamoyle de formule IIIa

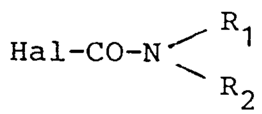

(IIIa)

ou bien

b) un carbamoylazolide de formule IIIb

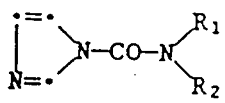

(IIIb)

ou bien, pour la préparation des composés de formule I dans laquelle $R_1$ ou $R_2$ représente l'hydrogène,

c) avec un isocyanate de formule IIIc

O = C = N-A (IIIc)

en présence d'une base, dans des solvants aprotoniques, sachant que, dans les formules ci-dessus, Hal représente un halogène, de préférence, le chlore, A a la signification de $R_1$ ou $R_2$ respectivement et $R_1$ et $R_2$, de même que X et Y, ont les significations indiquées en référence à la formule I.

**10.** Procédé de préparation des composés de formule I de la revendication 1, caractérisé en ce que l'on fait réagir :

un composé de formule II

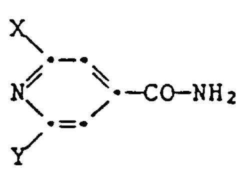

(II)

a) avec $(COCl)_2$ ou $CO(Cl)_2$, ce qui donne un isocyanate de formule IV

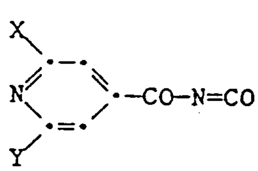

(IV),

qu'on fait réagir ensuite

b) avec une amine de formule V

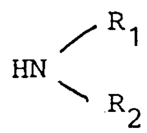

(V)

les différents stades de réaction étant réalisés dans des solvants inertes.

**11.** Produit pour protéger les végétaux contre l'attaque par les microorganismes, caractérisé en ce qu'il contient au moins un composant actif consistant en un composé selon la revendication 1 avec des véhicules et produits auxiliaires usuels.

**12.** Produit selon revendication 11, caractérisé en ce qu'il contient au moins un composant actif consistant en un composé selon les revendications 2 à 6.

**13.** Produit selon revendication 11, caractérisé en ce qu'il contient en tant que composant actif le composé de formule I de la revendication 7.

**14.** Produit selon revendication 11, caractérisé en ce qu'il contient en tant que composant actif le composé de formule I de la revendication 8.

**15.** Utilisation des composés selon revendication 1 pour la protection des végétaux contre l'attaque par des microorganismes phytopathogènes.

**16.** Utilisation des composés selon revendication 2 pour la protection des végétaux contre l'attaque par des microorganismes phytopathogènes.

**Revendications pour l'Etat contractant suivant : ES**

**1.** Procédé de préparation des composés de formule I

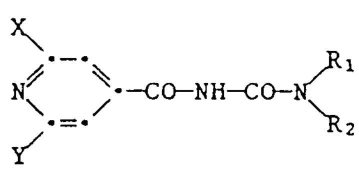

(I)

dans laquelle

- X représente l'hydrogène ou un halogène;
- Y représente un halogène;
- $R_1$, $R_2$ représentent chacun, indépendamment l'un de l'autre, l'hydrogène, un groupe alkyle en C1-C6, un groupe alkyle en C1-C6 substitué par des halogènes, des groupes cyano, alcoxy en C1-C4 ou COO-alkyle en C1-C3, un groupe alcényle en C3-C5, un groupe alcényle en C3-C5 substitué par des halogènes, un groupe alcynyle en C3-C5, un groupe alcynyle en C3-C5 substitué par des halogènes, un groupe cycloalkyle en C3-C7, un groupe cycloalkyle en C3-C7 substitué par des groupes alkyle en C1-C2, des halogènes, des groupes cyano ou COO-alkyle en C1-C3, un groupe phényle, benzyle, un groupe phényle ou benzyle substitué par des groupes alkyle en C1-C3, alcoxy en C1-C3 ou des halogènes; $R_1$ pouvant en outre représenter le groupe Q ou le groupe Q substitué par des groupes alkyle en C1-C3, cycloalkyle en C3-C7, alcoxy en C1-C3 ou des halogènes, Q pouvant être relié à l'atome d'azote par l'intermédiaire d'un groupe alkyle en C1-C2;
  ou bien $R_1$ et $R_2$ forment ensemble et avec l'atome d'azote voisin un hétérocycle de 3 à 7 chaînons qui peut contenir 1 ou 2 autres hétéroatomes tels que O, N ou S, et un groupe carbonyle, et qui peut être substitué par des groupes alkyle en C1-C3, des halogènes ou des groupes COO-alkyle en C1-C3;

Q représente un groupe furanne-2-yle, thiophène-2-yle, isoxazole-3-yle, isoxazole-5-yle, thiazole-2-yle, 1,2,4-thiadiazole-3-yle, 1,3,4-thiadiazole-2-yle, 2-, 3- ou 4-pyridyle, 2-, 3- ou 4-pyridyle substitué par des groupes alkyle en C1-C3, alcoxy en C1-C3, alkylthio en C1-C3 ou des halogènes; un groupe 2-, 4- ou 5-pyrimidyle ou un groupe 2-, 4- ou 5-pyrimidyle substitué par des groupes alkyle en C1-C3, alcoxy en C1-C3, alkylthio en C1-C3, des halogènes ou des groupes cyclopropyle, à l'exception du composé 2,6-dichloro-N-(((4-chlorophényl)-amino)-carbonyl)-4-pyridine-carboxamide,

caractérisé en ce que l'on fait réagir :
un isonicotinamide de formule II

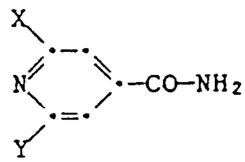

(II)

avec

a) un halogénure de carbamoyle de formule IIIa

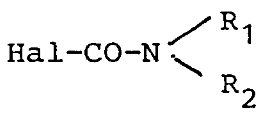

(IIIa)

ou bien

b) un carbamoylazolide de formule IIIb

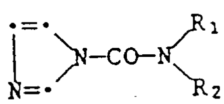

(IIIb)

ou bien, pour la préparation des composés de formule I dans laquelle $R_1$ ou $R_2$ représente l'hydrogène,

c) avec un isocyanate de formule IIIc

O = C = N-A (IIIc)

en présence d'une base, dans des solvants aprotoniques, sachant que, dans les formules ci-dessus, Hal représente un halogène, de préférence le chlore, A a la signification de $R_1$ ou $R_2$ respectivement et $R_1$ et $R_2$, de même que X et Y, ont les significations indiquées en référence à la formule I.

**2.** Procédé de préparation des composés de formule I

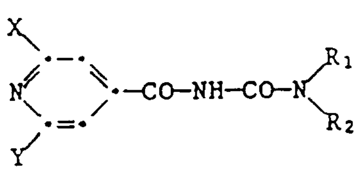

(I)

dans laquelle

X représente l'hydrogène ou un halogène;

Y représente un halogène;

$R_1$, $R_2$ représentent chacun, indépendamment l'un de l'autre, l'hydrogène, un groupe alkyle en C1-C6, un groupe alkyle en C1-C6 substitué par des halogènes, des groupes cyano ou COO-alkyle en C1-C3, un groupe alcényle en C3-C5, un groupe alcényle en C3-C5

substitué par des halogènes, un groupe alcynyle en C3-C5, un groupe alcynyle en C3-C5 substitué par des halogènes, un groupe cycloalkyle en C3-C7, un groupe cycloalkyle en C3-C7 substitué par des groupes alkyle en C1-C2, des halogènes, des groupes cyano ou COO-alkyle en C1-C3, un groupe benzyle, un groupe benzyle substitué par des groupes alkyle en C1-C3, alcoxy en C1-C3 ou des halogènes; et $R_1$ peut en outre représenter le groupe Q ou le groupe Q substitué par des groupes alkyle en C1-C3, cycloalkyle en C3-C7, alcoxy en C1-C3 ou des halogènes, Q pouvant être relié à l'atome d'azote par l'intermédiaire d'un groupe alkyle en C1-C2; ou bien $R_1$ et $R_2$ forment ensemble et avec l'atome d'azote voisin un hétérocycle de 3 à 7 chaînons qui peut en outre contenir 1 ou 2 autres hétéroatomes tels que O, N ou S et un groupe carbonyle et qui peut être substitué par des groupes alkyle en C1-C3; des halogènes ou des groupes COO-alkyle en C1-C3;

Q représente un groupe furanne-2-yle, thiophène-2-yle, isoxazole-3-yle, isoxazole-5-yle, thiazole-2-yle, 1,2,4-thiadiazole-3-yle, 1,3,4-thiadiazole-2-yle, 2-, 3- ou 4-pyridyle, 2-, 3- ou 4-pyridyle substitué par des groupes alkyle en C1-C3, alcoxy en C1-C3, alkylthio en C1-C3 ou des halogènes; un groupe 2-, 4- ou 5-pyrimidyle ou un groupe 2-, 4- ou 5-pyrimidyle substitué par des groupes alkyle en C1-C3, alcoxy en C1-C3, alkylthio en C1-C3, des halogènes ou des groupes cyclopropyle,

caractérisé en ce que l'on fait réagir :
un composé de formule II

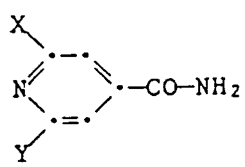

(II)

a) avec $(COCl)_2$ ou $CO(Cl)_2$, ce qui donne un isocyanate de formule IV

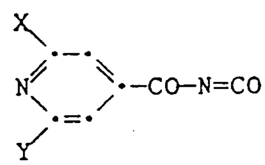

(IV),

qu'on fait réagir ensuite
b) avec une amine de formule V

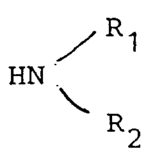

(V)

les différents stades de réaction étant réalisés dans des solvants inertes,
et X, Y, $R_1$ et $R_2$ ayant les significations indiquées en référence à la formule I.

**3.** Procédé selon revendication 1 ou 2, caractérisé en ce que l'on prépare des composés de formule I dans laquelle :

X représente l'hydrogène ou un halogène;

Y représente un halogène;

$R_1$, $R_2$ représentent chacun, indépendamment l'un de l'autre, l'hydrogène, un groupe alkyle en C1-C6, un groupe alkyle en C1-C6 substitué par des halogènes, des groupes cyano ou COO-alkyle en C1-C3, un groupe alcényle en C3-C5, un groupe alcényle en C3-C5 substitué par des halogènes, un groupe alcynyle en C3-C5, un groupe alcynyle en C3-C5 substitué par des halogènes, un groupe cycloalkyle en C3-C7, un groupe cycloalkyle en

C3-C7 substitué par des groupes alkyle en C1-C2, des halogènes, des groupes cyano ou COO-alkyle en C1-C3, un groupe benzyle, un groupe benzyle substitué par des groupes alkyle en C1-C3, alcoxy en C1-C3 ou des halogènes; et $R_1$ peut en outre représenter le groupe Q ou le groupe Q substitué par des groupes alkyle en C1-C3, cycloalkyle en C3-C7, alcoxy en C1-C3 ou des halogènes, Q pouvant être relié à l'atome d'azote par l'intermédiaire d'un groupe alkyle en C1-C2; ou bien $R_1$ et $R_2$ forment ensemble et avec l'atome d'azote voisin un hétérocycle de 3 à 7 chaînons qui peut en outre contenir 1 ou 2 autres hétéroatomes tels que O, N ou S et un groupe carbonyle et qui peut être substitué par des groupes alkyle en C1-C3, des halogènes ou des groupes COO-alkyle en C1-C3;

Q — représente un groupe furanne-2-yle, thiophène-2-yle, isoxazole-3-yle, isoxazole-5-yle, thiazole-2-yle, 1,2,4-thiadiazole-3-yle, 1,3,4-thiadiazole-2-yle, 2-, 3- ou 4-pyridyle, 2-, 3- ou 4-pyridyle substitué par des groupes alkyle en C1-C3, alcoxy en C1-C3, alkylthio en C1-C3 ou des halogènes; un groupe 2-, 4- ou 5-pyrimidyle ou un groupe 2-, 4- ou 5-pyrimidyle substitué par des groupes alkyle en C1-C3, alcoxy en C1-C3, alkylthio en C1-C3, des halogènes ou des groupes cyclopropyle.

**4.** Procédé selon revendication 1 ou 2, caractérisé en ce que l'on prépare des composés de formule I dans laquelle

X — représente l'hydrogène, le fluor, le chlore ou le brome;

Y — représente le fluor, le chlore ou le brome;

$R_1$ — représente un groupe alkyle en C1-C6, un groupe alkyle en C1-C6 substitué par le fluor, le chlore, des groupes cyano ou COO-alkyle en C1-C3, un groupe alcényle en C3-C5, un groupe alcényle substitué par le chlore, un groupe alcynyle en C3-C5, un groupe alcynyle en C3-C5 substitué par le chlore, un groupe cycloalkyle en C3-C7, un groupe cycloalkyle en C3-C7 substitué par un groupe COO-alcoxy en C1-C3, un groupe benzyle, un groupe benzyle substitué par des groupes alcoxy en C1-C3 ou des halogènes, ou un groupe Q:

$R_2$ — représente l'hydrogène, un groupe alkyle en C1-C6 ou un groupe alkyle en C1-C6 substitué par le fluor, le chlore, des groupes cyano ou COO-alkyle en C1-C3;

$R_1$ et $R_2$ — forment ensemble et avec l'atome d'azote voisin un hétérocycle de 4 à 7 chaînons qui peut contenir encore un autre atome d'azote ou un atome d'oxygène et qui peut être substitué par des groupes méthyle ou COO-alkyle en C1-C3;

Q — représente un groupe furanne-2-yle, thiophène-2-yle, isoxazole-3-yle ou isoxazole-5-yle, 2-, 3- ou 4-pyridyle, 2-, 3- ou 4-pyridyle substitué par des groupes alkyle en C1-C3, alcoxy en C1-C3 ou des halogènes, 2-, 4- ou 5-pyrimidyle ou 2-, 4- ou 5-pyrimidyle substitué par des groupes alkyle en C1-C3, alcoxy en C1-C3 ou des halogènes.

**5.** Procédé selon revendication 1 ou 2, caractérisé en ce que l'on prépare des composés de formule I dans laquelle :

X — représente l'hydrogène, le fluor, le chlore ou le brome;

Y — représente le fluor, le chlore ou le brome;

$R_1$ — représente un groupe alkyle en C1-C6, un groupe alkyle en C1-C6 substitué par des halogènes ou un groupe Q;

$R_2$ — représente l'hydrogène, un groupe alkyle en C1-C6, ou un groupe alkyle en C1-C6 substitué par le fluor, le chlore, des groupes cyano ou COO-alkyle en C1-C3;

$R_1$ et $R_2$ — forment ensemble et avec l'atome d'azote voisin un hétérocycle de 4 à 7 chaînons qui peut contenir un autre atome d'azote ou un atome d'oxygène et qui peut être substitué par des halogènes;

Q — représente un groupe thiazole-2-yle, 1,2,4-thiazole-3-yle, 1,3,4-thiadiazole-2-yle, 2-, 3- ou 4-pyridyle, 2-, 3- ou 4-pyridyle substitué par des groupes alkyle en C1-C3, alcoxy en C1-C3 ou des halogènes.

**6.** Procédé selon revendication 1 ou 2, caractérisé en ce que l'on prépare des composés de formule I dans laquelle :

X — représente l'hydrogène, le chlore ou le brome;

Y — représente le chlore ou le brome;

$R_1$ — représente un groupe alkyle en C1-C4, un groupe alkyle en C1-C4 substitué par le chlore ou des groupes COO-alkyle en C1-C3, un groupe alcényle en C3-C5, alcynyle

en C3-C5, cyclopropyle, cyclopropyle substitué par $COOCH_3$, benzyle, benzyle substitué par des groupes méthoxy ou le chlore; ou Q;

$R_2$ représente l'hydrogène, un groupe alkyle en C1-C4, un groupe alkyle en C1-C4 substitué par le chlore ou des groupes COO-alkyle en C1-C3;

$R_1$ et $R_2$ forment ensemble et avec l'atome d'azote voisin un hétérocycle à 5 ou 6 chaînons qui peut contenir un autre atome d'azote et un atome d'oxygène et qui peut être substitué par des groupes méthyle ou COO-alkyle en C1-C3;

Q représente un groupe furanne-2-yle, thiophène-2-yle, isoxazole-3-yle, isoxazole-5-yle, 2-, 3- ou 4-pyridyle, 2-, 3- ou 4-pyridyle substitué par des groupes alkyle en C1-C3, alcoxy en C1-C3, le chlore ou le brome; un groupe 2-, 4- ou 5-pyrimidyle ou un groupe 2-, 4- ou 5-pyrimidyle substitué par des groupes alkyle en C1-C3, alcoxy en C1-C3, le chlore ou le brome.

**7.** Procédé selon revendication 1 ou 2, caractérisé en ce que l'on prépare des composés de formule I dans laquelle :

X représente l'hydrogène, le chlore ou le brome;

Y représente le chlore ou le brome;

$R_1$ représente un groupe alkyle en C1-C4, un groupe alkyle en C1-C4 substitué par le fluor ou le chlore ou un groupe Q;

$R_2$ représente l'hydrogène, un groupe alkyle en C1-C4, un groupe alkyle en C1-C4 substitué par le chlore ou des groupes COO-alkyle en C1-C3;

$R_1$ et $R_2$ forment ensemble et avec l'atome d'azote voisin un hétérocycle à 5 ou 6 chaînons qui peut encore contenir un autre atome d'azote ou un atome d'oxygène et qui peut être substitué par le chlore ou le brome;

Q représente un groupe thiazole-2-yle, 1,2,4-thiadiazole-3-yle, 1,3,4-thiadiazole-2-yle, 2-, 3- ou 4-pyridyle, ou 2-, 3- ou 4-pyridyle substitué par des groupes méthyle, méthoxy, le fluor, le chlore ou le brome.

**8.** Procédé selon revendication 1 ou 2, caractérisé en ce que l'on prépare la N-(2,6-dichlorisonicotinoyl)-carbamoyl-pipéridine.

**9.** Procédé selon revendication 1 ou 2, caractérisé en ce que l'on prépare la N,N-diméthyl-N'-(2,6-dichlorisonicotinoyl)-urée.

**10.** Produit pour protéger les végétaux contre l'attaque par des microorganismes, caractérisé en ce qu'il contient, avec des véhicules et produits auxiliaires usuels, au moins un composant actif consistant en un composé de formule I

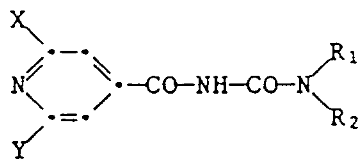

(I)

dans laquelle

X représente l'hydrogène ou un halogène;

Y représente un halogène;

$R_1$, $R_2$ représentent chacun, indépendamment l'un de l'autre, l'hydrogène, un groupe alkyle en C1-C6, un groupe alkyle en C1-C6 substitué par des halogènes, des groupes cyano, alcoxy en C1-C4 pu COO-alkyle en C1-C3; un groupe alcényle en C3-C5, un groupe alcényle en C3-C5 substitué par des halogènes, un groupe alcynyle en C3-C5, un groupe alcynyle en C3-C5 substitué par des halogènes, un groupe cycloalkyle en C3-C7, un groupe cycloalkyle en C3-C7 substitué par des groupes alkyle en C1-C2, des halogènes, des groupes cyano ou COO-alkyle en C1-C3, un groupe phényle, benzyle, un groupe phényle ou benzyle substitué par des groupes alkyle en C1-C3, alcoxy en C1-C3 ou des halogènes; $R_1$ pouvant en outre représenter le groupe Q ou le groupe Q substitué par des groupes alkyle en C1-C3, cycloalkyle en C3-C7, alcoxy en C1-C3 ou des halogènes,

Q pouvant être relié à l'atome d'azote par l'intermédiaire d'un groupe alkyle en C1-C2; ou bien $R_1$ et $R_2$ forment ensemble et avec l'atome d'azote voisin un hétérocycle de 3 à 7 chaînons qui peut contenir 1 ou 2 autres hétéroatomes tels que O, N ou S, et un groupe carbonyle, et qui peut être substitué par des groupes alkyle en C1-C3, des halogènes ou des groupes COO-alkyle en C1-C3;

Q représente un groupe furanne-2-yle, thiophène-2-yle, isoxazole-3-yle, isoxazole-5-yle, thiazole-2-yle, 1,2,4-thiadiazole-3-yle, 1,3,4-thiadiazole-2-yle, 2-, 3- ou 4-pyridyle, 2-, 3- ou 4-pyridyle substitué par des groupes alkyle en C1-C3, alcoxy en C1-C3, alkylthio en C1-C3 ou des halogènes; un groupe 2-, 4- ou 5-pyrimidyle ou un groupe 2-, 4- ou 5-pyrimidyle substitué par des groupes alkyle en C1-C3, alcoxy en C1-C3, alkylthio en C1-C3, des halogènes ou des groupes cyclopropyle.

**11.** Produit selon revendication 10, caractérisé en ce qu'il contient au moins un composant actif consistant en un composé de formule I

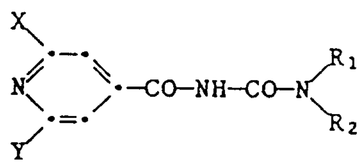

(I)

dans laquelle :

X représente l'hydrogène ou un halogène;

Y représente un halogène;

$R_1$, $R_2$ représentent chacun, indépendamment l'un de l'autre, l'hydrogène, un groupe alkyle en C1-C6, un groupe alkyle en C1-C6 substitué par des halogènes, des groupes cyano ou COO-alkyle en C1-C3, un groupe alcényle en C3-C5, un groupe alcényle en C3-C5 substitué par des halogènes, un groupe alcynyle en C3-C5, un groupe alcynyle en C3-C5 substitué par des halogènes, un groupe cycloalkyle en C3-C7, un groupe cycloalkyle en C3-C7 substitué par des groupes alkyle en C1-C2, des halogènes, des groupes cyano ou COO-alkyle en C1-C3, un groupe benzyle, un groupe benzyle substitué par des groupes alkyle en C1-C3, alcoxy en C1-C3 ou des halogènes; et $R_1$ peut en outre représenter le groupe Q ou le groupe Q substitué par des groupes alkyle en C1-C3, cycloalkyle en C3-C7, alcoxy en C1-C3 ou des halogènes, Q pouvant être relié à l'atome d'azote par l'intermédiaire d'un groupe alkyle en C1-C2; ou bien $R_1$ et $R_2$ forment ensemble et avec l'atome d'azote voisin un hétérocycle de 3 à 7 chaînons qui peut en outre contenir 1 ou 2 autres hétéroatomes tels que O, N ou S et un groupe carbonyle et qui peut être substitué par des groupes alkyle en C1-C3, des halogènes ou des groupes COO-alkyle en C1-C3;

Q représente un groupe furanne-2-yle, thiophène-2-yle, isoxazole-3-yle, isoxazole-5-yle, thiazole-2-yle, 1,2,4-thiadiazole-3-yle, 1,3,4-thiadiazole-2-yle, 2-, 3- ou 4-pyridyle, 2-, 3- ou 4-pyridyle substitué par des groupes alkyle en C1-C3, alcoxy en C1-C3, alkylthio en C1-C3 ou des halogènes; un groupe 2-, 4- ou 5-pyrimidyle ou un groupe 2-, 4- ou 5-pyrimidyle substitué par des groupes alkyle en C1-C3, alcoxy en C1-C3, alkylthio en C1-C3, des halogènes ou des groupes cyclopropyle.

**12.** Produit selon revendication 10, caractérisé en ce qu'il contient au moins un composant actif consistant en un composé de formule I dans laquelle :

X représente l'hydrogène, le fluor, le chlore ou le brome;

Y représente le fluor, le chlore ou le brome;

$R_1$ représente un groupe alkyle en C1-C6, un groupe alkyle en C1-C6 substitué par le fluor, le chlore, des groupes cyano ou COO-alkyle en C1-C3, un groupe alcényle en C3-C5, un groupe alcényle substitué par le chlore, un groupe alcynyle en C3-C5, un groupe alcynyle en C3-C5 substitué par le chlore, un groupe cycloalkyle en C3-C7, un groupe cycloalkyle en C3-C7 substitué par un groupe COO-alcoxy en C1-C3, un groupe benzyle, un groupe benzyle substitué par des groupes alcoxy en C1-C3 ou des halogènes, ou un groupe Q;

$R_2$ représente l'hydrogène, un groupe alkyle en C1-C6 ou un groupe alkyle en C1-C6

substitué par le fluor, le chlore, des groupes cyano ou COO-alkyle en C1-C3;

$R_1$ et $R_2$ forment ensemble et avec l'atome d'azote voisin un hétérocycle de 4 à 7 chaînons qui peut contenir encore un autre atome d'azote ou un atome d'oxygène et qui peut être substitué par des groupes méthyle ou COO-alkyle en C1-C3;

Q représente un groupe furanne-2-yle, thiophène-2-yle, isoxazole-3-yle ou isoxazole-5-yle, 2-, 3- ou 4-pyridyle, 2-, 3- ou 4-pyridyle substitué par des groupes alkyle en C1-C3, alcoxy en C1-C3 ou des halogènes; 2-, 4- ou 5-pyrimidyle ou 2-, 4- ou 5-pyrimidyle substitué par des groupes alkyle en C1-C3, alcoxy en C1-C3 ou des halogènes.

**13.** Produit selon revendication 10, caractérisé en ce qu'il contient au moins un composant actif consistant en un composé de formule I dans laquelle :

X représente l'hydrogène, le fluor, le chlore ou le brome;

Y représente le fluor, le chlore ou le brome;

$R_1$ représente un groupe alkyle en C1-C6, un groupe alkyle en C1-C6 substitué par des halogènes ou un groupe Q;

$R_2$ représente l'hydrogène, un groupe alkyle en C1-C6, ou un groupe alkyle en C1-C6 substitué par le fluor, le chlore, des groupes cyano ou COO-alkyle en C1-C3;

$R_1$ et $R_2$ forment ensemble et avec l'atome d'azote voisin un hétérocycle de 4 à 7 chaînons qui peut contenir un autre atome d'azote ou un atome d'oxygène et qui peut être substitué par des halogènes;

Q représente un groupe thiazole-2-yle, 1,2,4-thiazole-3-yle, 1,3,4-thiazole-2-yle, 2-, 3- ou 4-pyridyle, 2-, 3- ou 4-pyridyle substitué par des groupes alkyle en C1-C3, alcoxy en C1-C3 ou des halogènes.

**14.** Produit selon revendication 10, caractérisé en ce qu'il contient au moins un composant actif consistant en un composé de formule I dans laquelle :

X représente l'hydrogène, le chlore ou le brome;

Y représente le chlore ou le brome;

$R_1$ représente un groupe alkyle en C1-C4, un groupe alkyle en C1-C4 substitué par le chlore ou des groupes COO-alkyle en C1-C3, un groupe alcényle en C3-C5, alcynyle en C3-C5, cyclopropyle, cyclopropyle substitué par $COOCH_3$, benzyle, benzyle substitué par des groupes méthoxy ou le chlore; ou Q;

$R_2$ représente l'hydrogène, un groupe alkyle en C1-C4, un groupe alkyle en C1-C4 substitué par le chlore ou des groupes COO-alkyle en C1-C3;

$R_1$ et $R_2$ forment ensemble et avec l'atome d'azote voisin un hétérocycle à 5 ou 6 chaînons qui peut contenir un autre atome d'azote et un atome d'oxygène et qui peut être substitué par des groupes méthyle ou COO-alkyle en C1-C3;

Q représente un groupe furanne-2-yle, thiophène-2-yle, isoxazole-3-yle, isoxazole-5-yle, 2-, 3- ou 4-pyridyle, 2-, 3- ou 4-pyridyle substitué par des groupes alkyle en C1-C3, alcoxy en C1-C3, le chlore ou le brome; un groupe 2-, 4- ou 5-pyrimidyle ou un groupe 2-, 4- ou 5-pyrimidyle substitué par des groupes alkyle en C1-C3, alcoxy en C1-C3, le chlore ou le brome.

**15.** Produit selon revendication 10, caractérisé en ce qu'il contient au moins un composant actif consistant en un composé de formule I dans laquelle :

X représente l'hydrogène, le chlore ou le brome;

Y représente le chlore ou le brome;

$R_1$ représente un groupe alkyle en C1-C4, un groupe alkyle en C1-C4 substitué par le fluor ou le chlore ou un groupe Q;

$R_2$ représente l'hydrogène, un groupe alkyle en C1-C4, un groupe alkyle en C1-C4 substitué par le chlore ou des groupes COO-alkyle en C1-C3;

$R_1$ et $R_2$ forment ensemble et avec l'atome d'azote voisin un hétérocycle à 5 ou 6 chaînons qui peut encore contenir un autre atome d'azote ou un atome d'oxygène et qui peut être substitué par le chlore ou le brome;

Q représente un groupe thiazole-2-yle, 1,2,4-thiadiazole-3-yle, 1-,3,4-thiadiazole-2-yle, 2-, 3- ou 4-pyridyle, ou 2-, 3- ou 4-pyridyle substitué par des groupes méthyle, méthoxy, le fluor, le chlore ou le brome.

**16.** Produit selon revendication 10, caractérisé en ce qu'il contient en tant que composant actif le composé N-(2,6-dichlorisonicotinoyl)-carbamoyl-pipéridine.

**17.** Produit selon revendication 10, caractérisé en ce qu'il contient en tant que composant actif le composé N,N-diméthyl-N'-(2,6-dichlorisonicotinoyl)-urée.

**18.** Procédé pour protéger les végétaux contre l'attaque par des microorganismes phytopathogènes, caractérisé en ce que l'on applique sur la plante ou son habitat, en tant que substance active, un composé de formule I

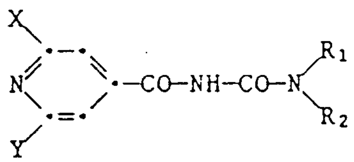

(I)

dans laquelle

X représente l'hydrogène ou un halogène;

Y représente un halogène;

$R_1$, $R_2$ représentent chacun, indépendamment l'un de l'autre, l'hydrogène, un groupe alkyle en C1-C6, un groupe alkyle en C1-C6 substitué par des halogènes, des groupes cyano, alcoxy en C1-C4 ou COO-alkyle en C1-C3, un groupe alcényle en C3-C5, un groupe alcényle en C3-C5 substitué par des halogènes, un groupe alcynyle en C3-C5, un groupe alcynyle en C3-C5 substitué par des halogènes, un groupe cycloalkyle en C3-C7, un groupe cycloalkyle en C3-C7 substitué par des groupes alkyle en C1-C2, des halogènes, des groupes cyano ou COO-alkyle en C1-C3, un groupe phényle, benzyle, un groupe phényle ou benzyle substitué par des groupes alkyle en C1-C3, alcoxy en C1-C3 ou des halogènes; $R_1$ pouvant en outre représenter le groupe Q ou le groupe Q substitué par des groupes alkyle en C1-C3, cycloalkyle en C3-C7, alcoxy en C1-C3 ou des halogènes, Q pouvant être relié à l'atome d'azote par l'intermédiaire d'un groupe alkyle en C1-C2;
ou bien $R_1$ et $R_2$ forment ensemble et avec l'atome d'azote voisin un hétérocycle de 3 à 7 chaînons qui peut contenir 1 ou 2 autres hétéroatomes tels que O, N ou S, et un groupe carbonyle, et qui peut être substitué par des groupes alkyle en C1-C3, des halogènes ou des groupes COO-alkyle en C1-C3;

Q représente un groupe furanne-2-yle, thiophène-2-yle, isoxazole-3-yle, isoxazole-5-yle, thiazole-2-yle, 1,2,4-thiadiazole-3-yle, 1,3,4-thiadiazole-2-yle, 2-, 3- ou 4-pyridyle, 2-, 3- ou 4-pyridyle substitué par des groupes alkyle en C1-C3, alcoxy en C1-C3, alkylthio en C1-C3 ou des halogènes; un groupe 2-, 4- ou 5-pyrimidyle ou un groupe 2-, 4- ou 5-pyrimidyle substitué par des groupes alkyle en C1-C3, alcoxy en C1-C3, alkylthio en C1-C3, des halogènes ou des groupes cyclopropyle,

**19.** Procédé pour protéger les végétaux contre l'attaque par des microorganismes phytopathogènes, caractérisé en ce que l'on applique sur la plante ou son habitat, en tant que substance active, un composé préparé selon une des revendications 2 à 9.